# EUROPÄISCHE PATENTSCHRIFT

(11) **EP 2 327 304 B1**
(45) Veröffentlichungstag und Bekanntmachung des Hinweises auf die Patenterteilung: **06.08.2014**
(21) Anmeldenummer: 10197164.6
(22) Anmeldetag: 19.03.2007
(51) Int. Cl.: A01N 43/08, A01N 43/40, A01N 43/78, A01P 5/00, A01P 7/00, A01P 7/02, A01P 7/04, C07D 277/32, C07D 277/22, C07D 417/12, C07D 213/04, C07D 213/26, C07D 213/61, C07D 405/12

(54) **Verwendung von substituierten Enaminocarbonylverbindungen zur Bekämpfung von tierischen Schädlingen**
Use of substituted enaminocarbonyl compounds for combating animal pests
Utilisation des composés énaminocarbonyle substitués pour combattre les parasites animaux

(30) Priorität: 31.03.2006 DE 102006015467
(43) Veröffentlichungstag der Anmeldung: 01.06.2011
(62) Teilanmeldung aus: 07723355.9
(73) Patentinhaber: Bayer CropScience AG, 40789 Monheim (DE)
(72) Erfinder: Jeschke, Peter, Dr., 51467 Bergisch Gladbach (DE); Velten, Robert, Dr., 40764 Langenfeld (DE); Schenke, Thomas, Dr., 51469 Bergisch Gladbach (DE); Schallner, Otto, Dr., 40789 Monheim (DE); Beck, Michael Edmund, Dr., 40789 Monheim (DE); Pontzen, Rolf, Dr., 42799 Leichlingen (DE); Malsam, Olga, Dr., 51503 Rösrath (DE); Reckmann, Udo, Dr., 51063 Köln (DE); Nauen, Ralf, Dr., 40764 Langenfeld (DE); Görgens, Ulrich, 40882 Ratingen (DE); Pitta, Leonardo, 51371 Leverkusen (DE); Müller, Thomas, Dr., 60323 Frankfurt am Main (DE); Arnold, Christian, Dr., 40764 Langenfeld (DE); Sanwald, Erich, Dr., 24159 Kiel (DE)

(56) Entgegenhaltungen:
- EP-A1- 0 539 588
- WO-A-2006/037475

## Beschreibung

Die vorliegende Anmeldung betrifft die Verwendung neuer substituierter Enaminocarbonylverbindungen zur Bekämpfung von Insekten, Spinnentieren und Nematoden.

Bestimmte substituierte Enaminocarbonylverbindungen sind bereits als insektizid wirksame Verbindungen bekannt geworden (vgl. EP 0539588 A1).

Es wurden nun neue Verbindungen der Formel (I) gefunden, 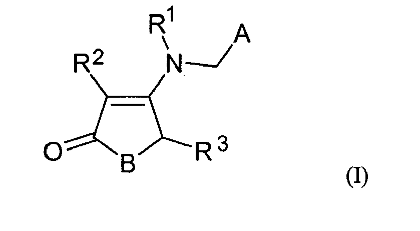 in welcher
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl,
B für Sauerstoff, Schwefel oder Methylen steht,
R¹ für Halogenalkyl, Halogenalkenyl, Halogencycloalkyl oder Halogencycloalkylalkyl steht,
R² für Wasserstoff oder Halogen steht und
R³ für Wasserstoff oder Alkyl steht.
Weiter wurde gefunden, dass man die neuen substituierten Verbindungen der Formel (I) erhält, wenn man

### a) Verbindungen der Formel (II)

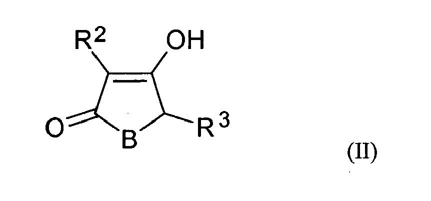

in welcher
B, R² und R³ die weiter oben genannten Bedeutungen haben
mit Verbindungen der Formel (III)

HN(R¹)-CH₂-A (III)

in welcher
A und R¹ die weiter oben genannten Bedeutungen haben,
gegebenenfalls in Gegenwart eines geeigneten Verdünnungsmittels und gegebenenfalls in Gegenwart eines sauren Hilfsmittels umsetzt (Verfahren 1), oder indem man

### b) Verbindungen der Formel (Ia)

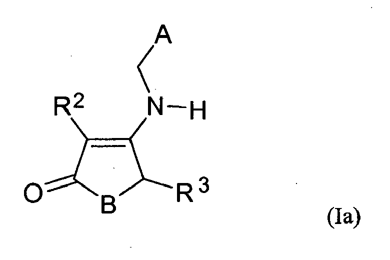

in welcher
A, B, R² und R³ die weiter oben genannten Bedeutungen haben
mit Verbindungen der Formel (IV)

E-R¹ (IV)

in welcher
R¹ die weiter oben genannten Bedeutungen hat und
E für eine geeignete Abgangsgruppe wie z.B. Halogen (insbesondere Brom, Chlor, Iod) oder O-Sulfonylalkyl und O-Sulfonylaryl (insbesondere 0-Mesyl, O-Tosyl) steht,
gegebenenfalls in Gegenwart eines geeigneten Verdünnungsmittels und gegebenenfalls in Gegenwart eines Säureakzeptors umsetzt (Verfahren 2), oder indem man

### c) Verbindungen der Formel (II)

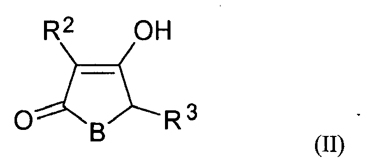

in welcher
B, R² und R³ die weiter oben genannten Bedeutungen haben,
in einem ersten Reaktionsschritt mit Verbindungen der Formel (V)

H₂N-R¹ (V)

in welcher
R¹ die weiter oben genannte Bedeutung hat
gegebenenfalls in Gegenwart eines geeigneten Verdünnungsmittels und gegebenenfalls in Gegenwart eines sauren Hilfsmittels umsetzt, und anschließend in einem zweiten Reaktionsschritt die gebildeten Verbindungen der Formel (VI) 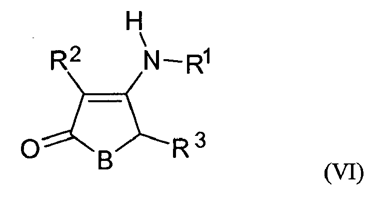 in welcher
B, R¹, R² und R³ die weiter oben genannten Bedeutungen haben, mit Verbindungen der Formel (VII)

E-CH₂-A (VII)

in welcher
E und A die weiter oben genannten Bedeutungen haben
gegebenenfalls in Gegenwart eines geeigneten Verdünnungsmittels und gegebenenfalls in Gegenwart eines Säureakzeptors umsetzt (Verfahren 3).

Schließlich wurde gefunden, das die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats-und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im Folgenden erläutert.
A steht bevorzugt für 6-Fluor-pyrid-3-yl, 6-Chlor-pyrid-3-yl, 6-Brom-pyrid-3-yl, 6-Methyl-pyrid-3-y 1 , 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl.
B steht bevorzugt für Sauerstoff oder Methylen.
R¹ steht bevorzugt für durch Fluor substituiertes C₁-C₅-Alkyl, C₂-C₅-Alkenyl, C₃-C_{S}-Cycloalkyl oder C₃-C₅-Cycloalkylalkyl.
R² steht bevorzugt für Wasserstoff oder Halogen (wobei Halogen insbesondere für Fluor oder Chlor steht),
R³ steht bevorzugt für jeweils Wasserstoff oder Methyl.
A steht besonders bevorzugt für den Rest 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl,
B steht besonders bevorzugt für Sauerstoff oder Methylen.
R¹ steht besonders bevorzugt für 2-Fluor-ethyl, 2,2-Difluor-ethyl, 2-Fluor-cyclopropyl.
R² steht besonders bevorzugt für Wasserstoff.
R³ steht besonders bevorzugt für Wasserstoff.
A steht ganz besonders bevorzugt für den Rest 6-Chlor-pyrid-3-yl, 6-Brom-pyrid-3-yl, oder 6-Chlor-1,4-pyridazin-3-yl,
B steht ganz besonders bevorzugt für Sauerstoff.
R¹ steht ganz besonders bevorzugt für 2,2-Difluor-ethyl.
R² steht ganz besonders bevorzugt für Wasserstoff.
R³ steht ganz besonders bevorzugt für Wasserstoff.

In einer hervorgehobenen Gruppe von Verbindungen der Formel (I) steht A für 6-Chlor-pyrid-3-yl

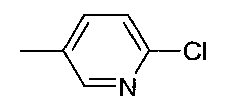

In einer weiteren hervorgehobenen Gruppe von Verbindungen der Formel (I) steht A für 6-Brom-pyrid-3-yl

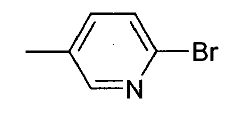

In einer weiteren hervorgehobenen Gruppe von Verbindungen der Formel (I) steht A für 6-Chlor-1,4-pyridazin-3-yl

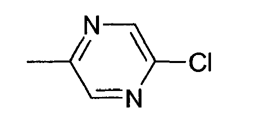

In einer weiteren hervorgehobenen Gruppe von Verbindungen der Formel (I) steht A für 2-Chlor-1,3-thiazol-5-yl

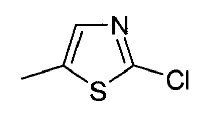

Im Folgenden ist eine weitere Gruppe bevorzugter Reste definiert, in welcher
A für Pyrid-3-yl, welches in 6-Position durch Fluor, Chlor, Brom, Methyl oder Trifluormethyl substituiert ist oder für 2-Chlor-pyrazin-5-yl oder für 2-Chlor-1,3-thiazol-5-yl steht,
B für Sauerstoff, Schwefel oder Methylen steht,
R¹ für Halogen-C₁₋₃alkyl, Halogen-C₂₋₃-alkenyl, Halogencyclopropyl steht (wobei Halogen insbesondere für Fluor oder Chlor steht),
R² für Wasserstoff oder Halogen steht und
R³ für Wasserstoff oder Methyl steht.
A steht bevorzugt für 6-Fluor-pyrid-3-yl, 6-Chlor-pyrid-3-yl, 6-Brom-pyrid-3-yl, 6-Trifluormethyl-pyrid-3-yl, 2-Chlor-pyrazin-5-yl oder 2-Chlor-1,3-thiazol-5-yl.
B steht bevorzugt für Sauerstoff oder Methylen.
R¹ steht bevorzugt für Difluormethyl, 2-Fluorethyl, 2,2-Difluorethyl, 2-Chlor-2-fluorethyl, 3-Fluor-*n*-propyl, 2-Fluor-vinyl, 3,3-Difluor-prop-2-enyl oder 3,3-Dichlor-prop-2-enyl.
R² steht bevorzugt für Wasserstoff oder Halogen (wobei Halogen insbesondere für Fluor oder Chlor steht).
R³ steht bevorzugt für jeweils Wasserstoff.
A steht besonders bevorzugt für den Rest 6-Chlor-pyrid-3-yl oder 6-Brom-pyrid-3-yl.
B steht besonders bevorzugt für Sauerstoff.
R¹ steht besonders bevorzugt für 2-Fluorethyl oder 2,2-Difluorethyl.
R² steht besonders bevorzugt für Wasserstoff.
R³ steht besonders bevorzugt für Wasserstoff.
A steht ganz besonders bevorzugt für den Rest 6-Chlor-pyrid-3-yl oder 6-Brom-pyrid-3-yl.
B steht ganz besonders bevorzugt für Sauerstoff.
R¹ steht ganz besonders bevorzugt für 2,2-Difluorethyl.
R² steht ganz besonders bevorzugt für Wasserstoff.
R³ steht ganz besonders bevorzugt für Wasserstoff..

In einer hervorgehobenen Gruppe von Verbindungen der Formel (I) stehen R³ für Wasserstoff, B für Sauerstoff und A für 6-Chlor-pyrid-3-yl

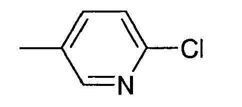

In einer weiteren hervorgehobenen Gruppe von Verbindungen der Formel (I) stehen R³ für Wasserstoff, B für Sauerstoff und A für 6-Brom-pyrid-3-yl

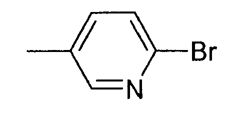

In einer weiteren hervorgehobenen Gruppe von Verbindungen der Formel (I) stehen R³ für Wasserstoff, B für Sauerstoff und A für 6-Fluor-pyrid-3-yl

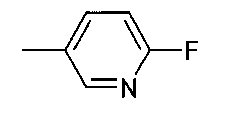

In einer weiteren hervorgehobenen Gruppe von Verbindungen der Formel (I) stehen R³ für Wasserstoff, B für Sauerstoff und A für 6-Trifluormethyl-pyrid-3-yl

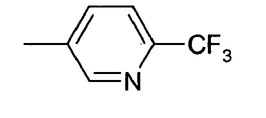

In einer weiteren hervorgehobenen Gruppe von Verbindungen der Formel (I) stehen R³ für Wasserstoff, B für Sauerstoff und A für 2-Chlor-1,3-thiazol-5-yl

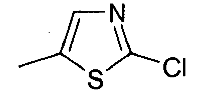

In einer weiteren hervorgehobenen Gruppe von Verbindungen der Formel (I) stehen R² und R³ für Wasserstoff, B für Sauerstoff und A für 6-Chlor-pyrid-3-yl

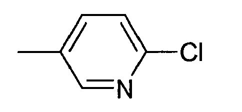

In einer weiteren hervorgehobenen Gruppe von Verbindungen der Formel (I) stehen R² und R³ für Wasserstoff, B für Sauerstoff und A für 6-Brom-pyrid-3-yl

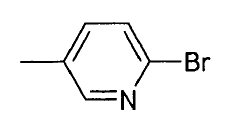

In einer weiteren hervorgehobenen Gruppe von Verbindungen der Formel (I) stehen R² und R³ für Wasserstoff, B für Sauerstoff und A für 6-Fluor-pyrid-3-yl

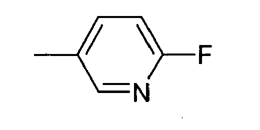

In einer weiteren hervorgehobenen Gruppe von Verbindungen der Formel (I) stehen R² und R³ für Wasserstoff, B für Sauerstoff und A für 6-Trifluormethyl-pyrid-3-yl

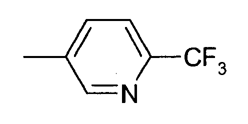

In einer weiteren hervorgehobenen Gruppe von Verbindungen der Formel (I) stehen R² und R³ für Wasserstoff, B für Sauerstoff und A für 2-Chlor-1,3-thiazol-5-yl

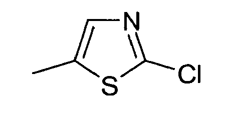

In einer weiteren hervorgehobenen Gruppe von Verbindungen der Formel (I) stehen R² und R³ für Wasserstoff, B für Methylen und A für 6-Chlor-pyrid-3-yl

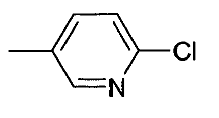

In einer weiteren hervorgehobenen Gruppe von Verbindungen der Formel (I) stehen R² und R³ für Wasserstoff, B für Methylen und A für 6-Brom-pyrid-3-yl

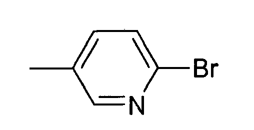

In einer weiteren hervorgehobenen Gruppe von Verbindungen der Formel (I) stehen R² und R³ für Wasserstoff, B für Methylen und A für 6-Fluor-pyrid-3-yl

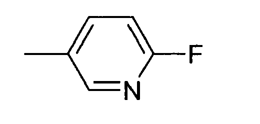

In einer weiteren hervorgehobenen Gruppe von Verbindungen der Formel (I) stehen R² und R³ für Wasserstoff, B für Methylen und A für 6-Trifluormethyl-pyrid-3-yl

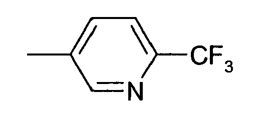

In einer weiteren hervorgehobenen Gruppe von Verbindungen der Formel (I) stehen R² und R³ für Wasserstoff, B für Methylen und A für 2-Chlor-1,3-thiazol-5-yl

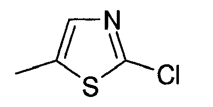

In einer weiteren hervorgehobenen Gruppe von Verbindungen der Formel (I) stehen R¹ für Difluormethyl, R² und R³ für Wasserstoff und B für Sauerstoff.

In einer weiteren hervorgehobenen Gruppe von Verbindungen der Formel (I) stehen R¹ für 2-Fluorethyl, R² und R³ für Wasserstoff und B für Sauerstoff.

In einer weiteren hervorgehobenen Gruppe von Verbindungen der Formel (I) stehen R¹ für 2,2-Difluorethyl, R² und R³ für Wasserstoff und B für Sauerstoff

In einer weiteren hervorgehobenen Gruppe von Verbindungen der Formel (I) stehen R¹ für Difluormethyl, R² und R³ für Wasserstoff und B für Methylen.

In einer weiteren hervorgehobenen Gruppe von Verbindungen der Formel (I) stehen R¹ für 2-Fluorethyl, R² und R³ für Wasserstoff und B für Methylen.

In einer weiteren hervorgehobenen Gruppe von Verbindungen der Formel (I) stehen R¹ für 2,2-Difluorethyl, R² und R³ für Wasserstoff und B für Methylen.

Die oben aufgeführten allgemeinen oder in Vorzugsbereiche aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Erfindungsgemäß ganz besonders bevorzugt werden Verbindungen der Formel (I), in welchen eine Kombination der vorstehend als ganz besonders bevorzugt aufgeführten Bedeutungen vorliegt.

Setzt man bei dem erfindungsgemäßen Verfahren 1 zur Herstellung der neuen Verbindungen der Formel (I) als Verbindung der Formel (II) beispielsweise die Tetronsäure ein und als Verbindung der Formel (III) das *N-*[6-Chlorpyridin-3-yl)methyl]-2,2-difluorethan-1-amin ein, so lässt sich das Herstellungsverfahren 1 durch folgendes Reaktionsschema I wiedergeben:

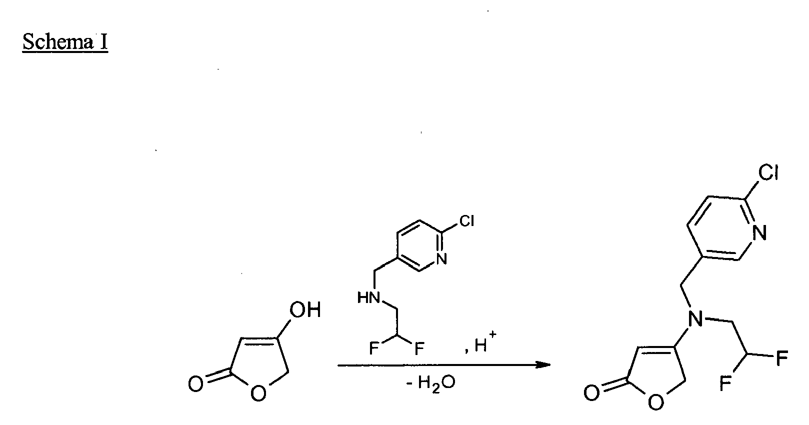

Die zur Durchführung des erfindungsgemäßen Verfahrens 1 als Ausgangsstoffe benötigten Verbindungen sind durch die Formel (II) allgemein definiert.

In dieser Formel (II) stehen B, R² und R³ vorzugsweise für diejenigen Reste, die bereits im Zusammenhang mit der Beschreibung der erfindungsgemäßen Stoffe der Formel (I) als bevorzugte Substituenten genannt werden.

Die Verbindungen der Formel (II) können z. T. kommerziell oder nach literaturbekannten Methoden erhalten werden (vgl. z. B. für Verbindungen der allgemeinen Formel II, in welcher B für Sauerstoff steht: Tetronsäuren (Said, A. Speciality Chemicals Magazine (1984), 4(4), 7-8; Rao, Y. S. Chem. Rev. (1976), 76, 625-694; Tejedor, D.; Garcia-Tellado, F. Org. Preparations and Procedures International (2004), 36, 35-59; Reviews); B für Schwefel steht: Thiotetronsäuren (Thomas, E. J. Special Publication - Royal Society of Chemistry (1988), 65 (Top. Med. Chem.), 284-307, Review), B für Methylen steht: Cyclopentan-1,3-dione (Schick, Hans; Eichhorn, Inge. Synthesis (1989), (7), 477-492, Review).

Die weiteren für die Durchführung des erfindungsgemäßen Verfahrens 1 als Ausgangsstoffe zu verwendenden Verbindungen sind allgemein durch die Formel (III) definiert.

In der Formel (III) haben A und R¹ die Bedeutungen, die bereits in Zusammenhang mit der Beschreibung der erfindungsgemäßen Stoffe der Formel (I) genannt wurden.

Die Verbindungen der Formel (III) können z. T. kommerziell oder nach literaturbekannten Methoden erhalten werden (vgl. z. B. S. Patai,,The Chemistry of Amino Group", Interscience Publishers, New York, 1968; Verbindungen der allgemeinen Formel (III), in welcher R¹ für Wasserstoff steht: primäre Amine, R¹ für Halogenalkyl, Halogenalkenyl oder Halogencycloalkyl steht: sekundäre Amine).

Die Verbindungen der Formel (III) lassen sich auch aus Verbindungen der Formel (VII) herstellen (vgl. Schema III weiter unten).

Die Verbindungen der Formel (VII) sind z. T. kommerziell erhältlich, teilweise bekannt und können nach bekannten Methoden erhalten werden (z. B. 2-Chlor-5-chlormethyl-1,3-thiazol: DE 3 631 538 (1988), EP 446 913 (1991), EP 780 384 (1997), EP 775 700 (1997), EP 794 180 (1997), WO 9 710 226 (1997); 6-Chlor-3-chlormethyl-pyridin: DE 3 630 046 A1 (1988), EP 373 464 A2 (1990), EP 373 464 A2 (1990), EP 393 453 A2 (1990), EP 569 947 A1 (1993); 6-Chlor-3-brommethyl-pyridin: I. Cabanal-Duvillard et al., Heterocycl. Commun. 5, 257-262 (1999); 6-Brom-3-chlormethyl-pyridin, 6-Brom-3-hydroxymethyl-pyridin: US-Pat. 5 420 270 A (1995); 6-Fluor-3-chlormethyl-pyridin: J. A. Pesti et al., J. Org. Chem. 65, 7718-7722 (2000); 6-Methyl-3-chlormethyl-pyridin: EP 302389 A2, E. v der Eycken et al., J. Chem. Soc., Perkin Trans 2 5, 928-937 (2002); 6-Trifluormethyl-3-chlormethyl-pyridin: WO 2004/082616 A2; 2-Chlor-5-chlormethyl-pyrazin: JP 05239034 A2).

Allgemeine Wege zur Herstellung von Verbindungen der Formel (VII) sind im Reaktionsschema II wiedergegeben. 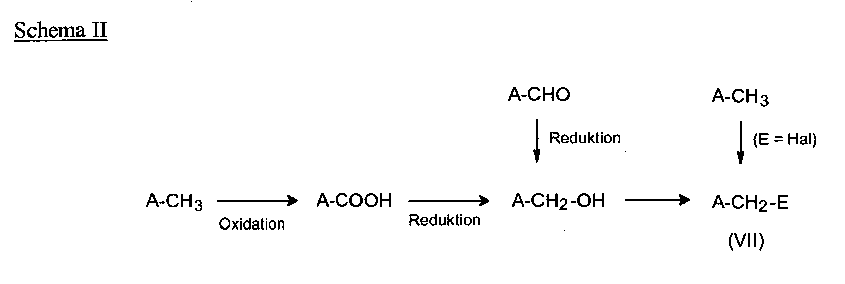
E = Hal, beispielsweise Chlor, Brom, lod; O-Tosyl, O-Mesyl,
A = wie oben definiert

Beispielsweise können die heterocyclischen Carbonsäuren (A-COOH) nach literaturbekannten Methoden in die entsprechenden heterocyclischen Hydroxymethylverbindungen (A-CH₂-OH) überführt werden, die anschließend nach literaturbekannten Methoden zu aktivierten heterocyclischen Hydroxymethylverbindungen (A-CH₂-E, E = OTosyl, OMesyl) oder heterocyclischen Halogenmethylverbindungen (A-CH₂-E, E = Hal) umgesetzt werden. Letztere können auch aus entsprechenden Methylgruppe-haltigen Heterocyclen (A-CH₃) unter Verwendung von geeigneten literaturbekannten Halogenierungsmitteln erhalten werden.

Zur Darstellung von Verbindungen der Formel (III) ist es vorteilhaft, dass man beispielsweise Verbindungen der Formel (VII), in der A und E die weiter oben genannte Bedeutung haben, mit Verbindungen der Formel (V), in der R¹ die weiter oben genannte Bedeutung hat, gegebenenfalls in Gegenwart von Verdünnungsmitteln und gegebenenfalls in Gegenwart von den im Herstellungs-verfahren 2 genannten basischen Reaktionshilfsmitteln umsetzt (vgl. *N*-Alkylierung, Schema III). 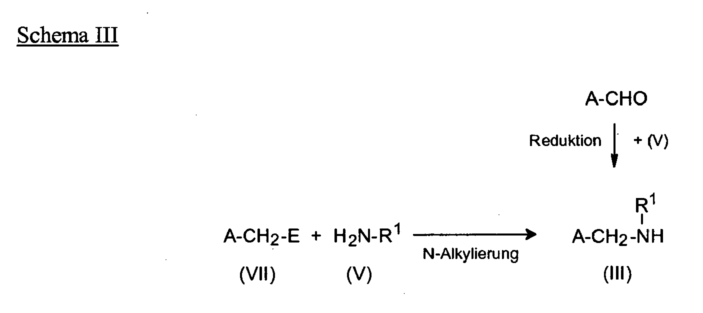
E = Hal, beispielsweise Chlor, Brom, lod; O-Tosyl, O-Mesyl,
A = wie oben definiert

Die Verbindungen der Formel (V) können z. T. kommerziell (vgl. z. B. 2-Fluorethylamin oder 2,2-Difluorethylamin) oder nach literaturbekannten Methoden erhalten werden (vgl. z. B. 3-Fluor-*n-*propylamin: US 6252087 B1; 3,3-Difluor-prop-2-enylamin Hydrochlorid: WO 2001/007414 A1; 3,3-Dichlor-prop-2-enylamin: DE 2747814).

Alternativ und in bestimmten Fällen ist aber auch eine Herstellung von Verbindungen der Formel (III) aus den entsprechenden Aldehyden (A-CHO) und Verbindungen der Formel (V) mittels reduktiver Aminierung möglich (vgl. Houben-Weyl, Methoden der Organischen Chemie, Bd. XI/1, Georg Thieme Verlag Stuttgart, S. 602). Die Aldehyde (A-CHO) sind z. T. kommerziell erhältlich (vgl. z. B. 6-Chlor-nicotinaldehyd, 6-Fluor-nicotinaldehyd, 6-Brom-nicotinaldehyd, 2-Chlor-1,3-thiazol-5-carbaldehyd) oder können nach literaturbekannten Methoden erhalten werden (vgl. z. B. 6-Methyl-nicotinaldehyd: EP 104876 A2; 2-Chlor-pyrazin-5-caboxaldehyd: DE 3314196 A1).

Im Allgemeinen ist es vorteilhaft, das erfindungsgemäße Herstellungsverfahren 1 in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens 1 kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether wie Ethylpropylether, Methyl-tert-butylether, *n-*Butylether, Anisol, Phenetol, Cyclohexylmethylether, Dimethylether, Diethylether, Diproplether, Diisopropylether, Di-*n*-butylether, Diisobutylether, Diisoamylether, Ethylenglycoldimethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, *N-*Methyhmorpholin, Pyridin und Tetramethylendiamin; Nitrokohlenwasserstoffe wie Nitromethan, Nitroethan, Nitropropan, Nitrobenzol. Chlornitrobenzol, o-Nitrotoluol; Nitrile wie Acetonitril, Propionitril, Butyronitril, Isobutyronitril, Benzonitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe; beispielsweise sogenannte White Spirits mit Komponenten mit Siedepunkten im Bereich beispielsweise von 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, *N-*Methyl-formamid, *N,N-*Dimethyl-formamid, *N,N-*Dipropyl-formamid, *N,N-*Dibutyl-formamid, *N-*Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N-*Formylpiperidin, *N,N'*-1,4-Diformyl-piperazin; Ketone wie Aceton, Acetophenon, Methylethylketon, Methylbutylketon.

Man kann in das erfindungsgemäße Verfahren auch Gemische der genannten Lösungs- und Verdünnungsmittel einsetzen.

Bevorzugte Verdünnungsmittel zur Durchführung des erfindungsgemäßen Verfahrens sind aromatische Kohlenwasserstoffe wie Benzol, Toluol, Chlorbenzol, Brombenzol, Nitrobenzol oder Xylol, insbesondere Benzol und Toluol.

Die Herstellung von Verbindungen der Formel (I) nach dem Herstellungsverfahren 1 wird durchgeführt, indem man Verbindungen der Formel (II) in Gegenwart von Verbindungen der Formel (III), gegebenenfalls in Gegenwart eines sauren Hilfsmittels und gegebenenfalls in einem der angegebenen Verdünnungsmittel umsetzt.

Die Reaktionsdauer beträgt im Allgemeinen 10 Minuten bis 48 Stunden. Die Umsetzung erfolgt bei Temperaturen zwischen -10°C und +200°C, bevorzugt zwischen +10°C und 180°C, besonders bevorzugt zwischen 60°C und 140°C. Man arbeitet bevorzugt unter Reaktionsbedingungen, die das Abscheiden oder Entfernen von Wasser ermöglichen, beispielsweise unter Zuhilfenahme eines Wasserabscheiders.

Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des erfindungsgemäßen Verfahrens 1 setzt man pro Mol Verbindung der allgemeinen Formel (II) im Allgemeinen 0,5 bis 4,0 Mol, vorzugsweise 0,7 bis 3,0 Mol, besonders bevorzugt 1,0 bis 2,0 Mol an Aminoverbindung der allgemeinen Formel (III) ein.

Weiterhin kann man zur Durchführung des erfindungsgemäßen Verfahrens 1 im Allgemeinen katalytische Mengen eines sauren Hilfsmittels hinzusetzen.

Als saure Hilfsmittel kommen beispielsweise *p*-Toluolsulfonsäure oder Essigsäure in Frage.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Setzt man bei dem erfindungsgemäßen Verfahren 2 zur Herstellung der neuen Verbindungen der Formel (I) als Verbindung der Formel (Ia) beispielsweise 4-[[(6-Chlor-pyridin-3-yl)methyl]-amino]furan-2(5H)-on und als Verbindung der Formel (IV) das 3-Brom-1,1-dichlor-prop-1-en ein, so lässt sich das Herstellungsverfahren 2 durch folgendes Reaktionsschema IV wiedergeben:

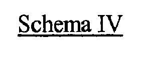

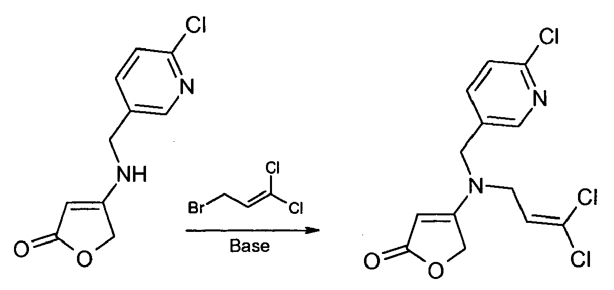

Die zur Durchführung des erfindungsgemäßen Verfahrens 2 als Ausgangsstoffe benötigten Verbindungen sind durch die Formel (la) allgemein definiert.

In dieser Formel (la) stehen A, B, R² und R³ vorzugsweise für diejenigen Reste, die bereits im Zusammenhang mit der Beschreibung der erfindungsgemäßen Stoffe der Formel (I) als bevorzugte Substituenten genannt werden.

Die Verbindungen der Formel (la) können nach dem weiter oben beschriebenen Herstellverfahren 1 erhalten werden, beispielsweise durch Reaktion von Verbindungen der Formel (II) mit Verbindungen der Formel (III), in welcher R¹ für Wasserstoff steht.

Die insbesondere für die Durchführung des erfindungsgemäßen Verfahrens 2 als Ausgangsstoffe zu verwendenden Verbindungen sind allgemein durch die Formel (IV) definiert.

In der Formel (IV) haben E und R¹ die Bedeutung die bereits im Zusammenhang mit der Beschreibung der erfindungsgemäßen Stoffe der allgemeinen Formel (I) für Substituenten genannt wurde.

Die Verbindungen der Formel (IV) können z. T. kommerziell erhalten werden (vgl. z. B. Chlordifluormethan, 1-Brom-2-fluoretan, 2-Brom-1,1-difluorethan, 2-Brom-1-chlor-1-fluorethan, 1-Brom-3-fluorpropan, 3-Brom-1,1-difluor-prop-1-en, oder nach literaturbekannten Methoden erhalten werden (vgl. z. B. 3-Brom-1,1-dichlor-prop-1-en: WO 8800183 A1 (1988); Verbindungen der allgemeinen Formel IV in welcher E für Halogen wie Chlor, Brom und Iod steht: Houben-Weyl, Methoden der Organischen Chemie, Bd, V/3, Georg Thieme Verlag Stuttgart, S. 503 und Bd. V/4 S. 13, 517; E¹ für Mesylat steht: Crossland, R. K., Servis, K. L. J. Org. Chem. (1970), 35, 3195; E für Tosylat steht: Roos, A. T. et al., Org. Synth., Coll. Vol. I, (1941), 145; Marvel, C. S., Sekera, V. C. Org. Synth., Coll. Vol. III, (1955), 366.

Im Allgemeinen ist es vorteilhaft, das erfindungsgemäße Herstellungsverfahren 2 in Gegenwart von Verdünnungsmitteln und in Gegenwart von basischen Reaktionshilfsmitteln durchzuführen.

Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens 2 kommen alle inerten organischen Lösungsmittel in Frage.

Bevorzugte Verdünnungsmittel zur Durchführung des erfindungsgemäßen Verfahrens 2 sind Ether wie Methy1-*tert*-butylether, *n*-Butylether, Anisol, Phenetol, Cyclohexylmethylether, Diisopropylether, Diisobutylether, Diisoamylether, Ethylenglycoldimethylether, Tetrahydrofuran, Dioxan, Dichlordiethylether und Polyether des Ethylenoxids und/oder Propylenoxids, Amide wie Hexamethylenphosphorsäuretriamid, Formamid, *N-*Methyl-formamid, *N,N-*Dimethyl-formamid, *N,N-*Dipropyl-formamid, *N,N-*Dibutyl-formamid, Benzol, Toluol, Chlorbenzol, Brombenzol, Nitrobenzol, Xylol; Ketone wie Aceton, Acetophenon, Methylethylketon oder Methylbutylketon.

Selbstverständlich kann man in das erfindungsgemäße Verfahren auch Gemische der genannten Lösungs- und Verdünnungsmittel einsetzen.

Bevorzugte Verdünnungsmittel zur Durchführung des erfindungsgemäßen Verfahrens sind jedoch Ether wie Methyl-*tert*-butylether oder cyclische Ether wie Tetrahydrofuran und Dioxan, Amide wie *N,N-*Dimethyl-formamid, aromatische Kohlenwasserstoffe wie Benzol oder Toluol; Ketone wie Aceton, Methylethylketon oder Methylbutylketon.

Als basische Reaktionshilfsmittel zur Durchführung des erfindungsgemäßen Verfahrens 2 können alle geeigneten Säurebindemittel eingesetzt werden wie Amine, insbesondere tertiäre Amine sowie Alkali- und Erdalkaliverbindungen.

Beispielhaft seien dafür erwähnt die Hydroxide, Hydride, Oxide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triaza-bicyclo(4.4.0)dec-5-en (MTBD); Diazabicyclo(4.3.0)nonen (DBN), Diazabicyclo (2.2.2)octan (DABCO), 1,8-Diazabicyclo-(5.4.0)undecen (DBU), Cyclohexyltetrabutyl-guanidin (CyTBG), Cyclohexyltetramethylguanidin (CyTMG), *N,N,N,N*-Tetramethyl-1,8-naphthalindiamin, Pentamethylpiperidin, tertiäre Amine wie Triethylamin, Trimethylamin, Tribenzylamin, Triisopropylamin, Tributylamin, Tricyclohexylamin, Triamylamin, Trihexylamin, *N,N-*Dimethylanilin, *N,N-*Dimethyl-toluidin, N,N Dimethyl p-aminopyridin, *N-*Methyl-pyrrolidin, *N-*Methyl-piperidin, *N-*Methyl-imidazol, *N-*Methyl-pyrazol, *N-*Methyl-morpholin, *N-*Methyl-hexamethylendiamin, Pyridin, 4-Pyrrolidinopyridin, 4-Dimethylaminopyridin, Chinolin, a-Picolin, β-Picolin, Isochinolin, Pyrimidin, Acridin, *N,N,N',N'*-Tetramethylendiamin, *N,N,N',N'*-Tetraethylendiamin, Chinoxalin, *N*-Propyl-diisopropylamin, *N*-Ethyldiisopropylamin, *N,N'*-Dimethyl-cyclohexylamin, 2,6-Lutidin, 2,4-Lutidin oder Triethyldiamin.

Vorzugsweise finden Hydride des Lithiums oder Natriums Verwendung.

Die Reaktionsdauer beträgt im Allgemeinen 10 Minuten bis 48 Stunden. Die Umsetzung erfolgt bei Temperaturen zwischen -10°C und +200°C, bevorzugt zwischen +10°C und 180°C, besonders bevorzugt zwischen 60°C und 140°C. Es kann grundsätzlich unter Normaldruck gearbeitet werden. Vorzugsweise arbeitet man bei Normaldruck oder bei Drucken bis zu 15 bar und gegebenenfalls unter Schutzgasatmosphäre (Stickstoff, Helium oder Argon).

Zur Durchführung des erfindungsgemäßen Verfahrens 2 setzt man pro Mol Verbindung der Formel (II) im Allgemeinen 0,5 bis 4,0 Mol, vorzugsweise 0,7 bis 3,0 Mol, besonders bevorzugt 1,0 bis 2,0 Mol an Alkylierungsmittel der Formel (IV) ein.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Setzt man bei dem erfindungsgemäßen Verfahren 3 zur Herstellung der neuen Verbindungen der Formel (I) in einem ersten Reaktionsschritt als Verbindungen der Formel (II) beispielsweise die Tetronsäure ein und als Verbindung der Formel (V) das 2-Fluorethylamin ein, und in einem zweiten Reaktionsschritt als entstandene Verbindung der Formel (VI) das 4-[(2-Fluorethyl)amino]furan-2(5H)-on, welches mit Verbindungen der Formel (VII), beispielsweise 2-Chlor-5-(chlormethyl)pyridin *N*-alkyliert wird, so lässt sich das Herstellungsverfahren 3 durch folgendes Reaktionsschema V wiedergeben:

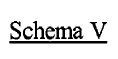

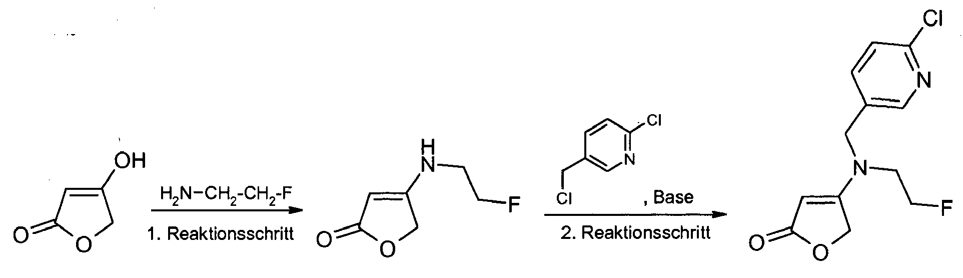

Die zur Durchführung des erfindungsgemäßen Verfahrens 3 als Ausgangsstoffe benötigten Verbindungen sind durch die Formel (II) allgemein definiert und bereits im weiter oben genannten Verfahren 1 näher beschrieben.

Die weiterhin für die Durchführung des erfindungsgemäßen Verfahrens 3 als Ausgangsstoffe zu verwendenden Verbindungen sind allgemein durch die Formel (V) definiert.

In der Formel (V) hat R¹ die Bedeutung, die bereits im Zusammenhang mit der Beschreibung der erfindungsgemäßen Stoffe der Formel (I) genannt wurde.

Die Aminoverbindungen der Formel (V) sind allgemein definiert und können in vielen Fällen z. T. kommerziell (vgl. z. B. 2-Fluorethylamin oder 2,2-Difluorethylamin) oder in an sich bekannter Weise nach der "Leuckart-Wallach-Reaktion (z. B. 2-Fluor-ethylamin: US-Pat. 4030994 (1977); Verbindungen der Formel (V) in welcher R¹ für Alkyl steht, primäre Amine: vgl. z. B. Houben-Weyl, Methoden der Organischen Chemie, Bd. XI/1, 4. Aufl. 1957, Georg Thieme Verlag Stuttgart, S. 648; M. L. Moore in "The Leuckart Reaction" in: Organic Reactions, Bd. 5, 2. Aufl. 1952, New York, John Wiley & Sons, Inc. London) erhalten werden (vgl. z. B. auch 3-Fluor-*n*-propylamin: US 6252087 B1; 3,3-Difluor-prop-2-enylamin Hydrochlorid: WO 2001/007414 A1; 3,3-Dichlor-prop-2-enylamin: DE 2747814); 2-Chlor-2-fluor-cyclopropylamin, 2,2-Dichlor-cyclopropylamin: K. R. Gassen, B. Baasner, J. Fluorine Chem. 49, 127-139, 1990).

Alternativ können bestimmte Aminoverbindungen der Formel (Va), in welcher R¹ für CH₂-R*'* (R'= halogenhaltiger Rest; Halogen = Fluor oder Chlor) steht, auch durch Reduktion von halogenierten Carbonsäureamiden (VIII) in Gegenwart geeigneter Reduktionsmittel erhalten werden (Reaktionsschema VI). 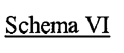 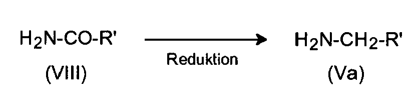
R' = halogenhaltiger Rest

Als bevorzugtes Reduktionsmittel eignet sich beispielsweise der bekannte Boran-Dimethylsulfid Komplex (vgl. auch die Herstellung von 2-Chlor-2-fluorethan-1-amin aus kommerziell erhältlichem 2-Chlor-2-fluoracetamid).

Die weiterhin für die Durchführung des erfindungsgemäßen Verfahrens 3 als Ausgangsstoffe zu verwendenden Verbindungen sind allgemein durch die Formel (VII) definiert.

In der Formel (VII) haben E und A die Bedeutung, die bereits im Zusammenhang mit der Beschreibung der erfindungsgemäßen Stoffe der Formel (I) genannt wurde.

Die Verbindungen der allgemeinen Formel (VII) sind wie bereits weiter oben erwähnt z. T. kommerziell erhältlich, teilweise bekannt oder können nach bekannten Methoden erhalten werden.

Im Allgemeinen ist es vorteilhaft, den ersten Reaktionsschritt des erfindungsgemäßen Herstellungsverfahrens 3 in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung des erfindungsgemäßen Verfahrens 3 kommen alle inerten organischen Lösungsmittel in Frage.

Bevorzugte Verdünnungsmittel zur Durchführung des ersten Reaktionsschritts des erfindungsgemä-βen Verfahrens 3 sind aromatische Kohlenwasserstoffe wie Benzol, Toluol, Chlorbenzol, Brombenzol, Nitrobenzol oder Xylol, insbesondere aber Benzol und Toluol.

Im zweiten Reaktionsschritt werden die Verbindungen der Formel (VI) mit Verbindungen der Formel (VII) N-alkyliert.

Im Allgemeinen ist es vorteilhaft, den zweiten Reaktionsschritt des erfindungsgemäßen Herstellungsverfahrens 3 in Gegenwart von Verdünnungsmitteln und in Gegenwart von basischen Reaktionshilfsmitteln wie beispielsweise Natriumhydrid durchzuführen.

Als Verdünnungsmittel für diesen Reaktionsschritt kommen beispielsweise Ether wie Tetrahydrofuran oder Dioxan in Frage.

Verdünnungsmittel werden vorteilhaft in einer solchen Menge eingesetzt, dass das Reaktionsgemisch während des ganzen Verfahrens gut rührbar bleibt.

Die Reaktionsdauer beträgt im Allgemeinen 10 Minuten bis 48 Stunden.

Die Umsetzung erfolgt bei Temperaturen zwischen -10°C und +200°C, bevorzugt zwischen +10°C und 180°C, besonders bevorzugt zwischen 60°C und 140°C. Man arbeitet bevorzugt unter Reaktionsbedingungen, die das Abscheiden oder Entfernen von Wasser ermöglichen, beispielsweise unter Zuhilfenahme eines Wasserabscheiders.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Zur Darstellung der Verbindungen der Formel (I) in der R² für einen 2-Fluor-vinyl Rest steht, werden erfindungsgemäß Verbindungen der Formel (I) in der R² für einen 2-Chlor-2-fluorethyl Rest steht, in Gegenwart eines basischen Hilfsmitteln gemäß dem Reaktionsschema (VII) dehydrohalogeniert (d.h. formal HC1 Abspaltung). Die Reaktionsprodukte können in Form von geometrischen Isomeren, beispielsweise *(E)*- und *(Z)*-Isomeren (bzw. *trans-* und *cis*-Isomeren) vorliegen.

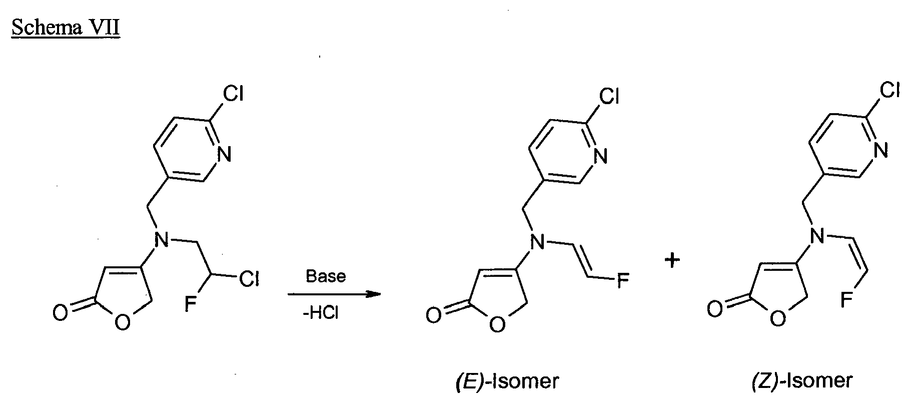

In den zur Dehydrohalogenierung als Ausgangsstoffe benötigten Verbindungen der Formel (I) haben A, B, R² und R³ die weiter oben genannte Bedeutung, der Substituent R¹ hat die Bedeutung von 2-Chlor-2-fluorethyl.

Diese Verbindungen der Formel (I) können nach den weiter oben genannten Herstellverfahren 1 bis 3 erhalten werden.

Im Allgemeinen ist es vorteilhaft, die Dehydrohalogenierung in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung der C-Alkylierung kommen alle unter den Reaktionsbedingungen inerten organischen Lösungsmittel in Frage.

Bevorzugte Verdünnungsmittel zur Durchführung der Dehydrohalogenierung sind Alkohole wie Methanol, Ethanol, Isopropanol, Butanol; Man kann in das erfindungsgemäße Verfahren auch Gemische der genannten Lösungs- und Verdünnungsmittel einsetzen.

Bevorzugte Verdünnungsmittel zur Durchführung des erfindungsgemäßen Verfahrens sind Alkohole wie Ethanol oder Butanol.

Die Dehydrohalogenierung von Verbindungen der Formel (I) wird durchgeführt, indem man diese mit Verbindungen der allgemeinen Formel (I) in Gegenwart von basischen Reaktionshilfsmitteln umsetzt.

Im Allgemeinen ist es vorteilhaft, als basische Reaktionshilfsmittel Alkalimetallhydroxyde wie beispielsweise Natriumhydroxid oder Kaliumhydroxid zu verwenden.

Die Reaktionsdauer beträgt im Allgemeinen 10 Minuten bis 48 Stunden.

Die Umsetzung erfolgt bei Temperaturen zwischen -100°C und +80°C, bevorzugt zwischen -20°C und 50°C, besonders bevorzugt bei Raumtemperatur.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen. Der Nachweis der geometrischen Isomere, beispielsweise der *(E)-*und *(Z)*-Isomere, gelingt mittels bekannter analytischer Methoden. Beispielsweise liegt im o. g. Beispiel ein *(E*/*Z)*-Isomerengemisch im Verhältnis von (15:85) vor (vgl. auch die Herstellungsbeispiele).

Zur Darstellung der Verbindungen der Formel (I) in der R³ für Alkyl steht werden erfindungsgemäß Verbindungen der Formel (I) in der R³ für Wasserstoff steht, mit Verbindungen der Formel (IV) in Gegenwart von basischen Hilfsmitteln gemäß dem Reaktionsschema (VIII) umgesetzt.

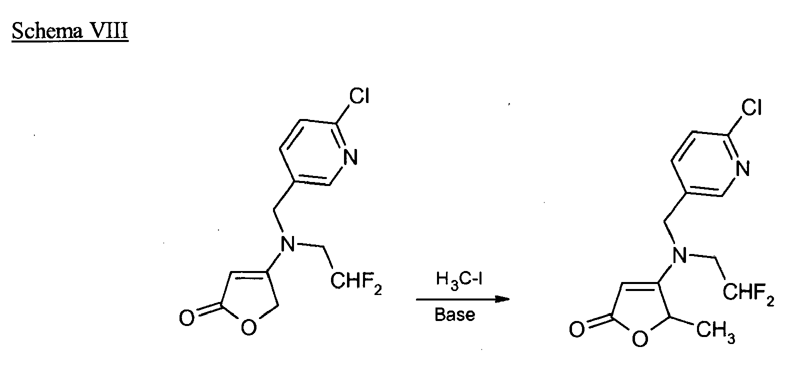

In den für die C-Alkylierung als Ausgangsstoffe benötigten Verbindungen der Formel (I) haben A, B, R² und R³ die weiter oben genannte Bedeutung, der Substituent R¹ hat die Bedeutung von Wasserstoff.

Diese Verbindungen der Formel (I) können nach den weiter oben genannten Herstellverfahren 1 bis 3 erhalten werden.

Im Allgemeinen ist es vorteilhaft, die C-Alkylierung in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung der C-Alkylierung kommen alle inerten organischen Lösungsmittel in Frage.

Bevorzugte Verdünnungsmittel zur Durchführung der C-Alkylierung sind Ether wie Methyl-*tert-*butylether, *n*-Butylether, Anisol, Phenetol, Cyclohexylmethylether, Diisopropylether, Diisobutylether, Diisoamylether, Ethylenglycoldimethylether, Tetrahydrofuran, Dioxan, Dichlordiethylether und Polyether des Ethylenoxids und/oder Propylenoxids.

Man kann in das erfindungsgemäße Verfahren auch Gemische der genannten Lösungs- und Verdünnungsmittel einsetzen.

Bevorzugte Verdünnungsmittel zur Durchführung des erfindungsgemäßen Verfahrens sind Ether wie Methyl-*tert*-butylether oder cyclische Ether wie Tetrahydrofuran und Dioxan.

Die C-Allcylierung wird durchgeführt, indem man geeignete Ausgangsverbindungen der Formel I mit Verbindungen der Formel (IV) in Gegenwart von basischen Reaktionshilfsmitteln umsetzt.

Die Reaktionsdauer beträgt im Allgemeinen 10 Minuten bis 48 Stunden.

Die Umsetzung erfolgt bei Temperaturen zwischen -100°C und +20°C, bevorzugt zwischen -90°C und 10°C, besonders bevorzugt zwischen -80°C und 0°C.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Zur Darstellung der Verbindungen der Formel (I) in der R² für Halogen steht, können alternativ auch Verbindungen der Formel (I) in der R² für Wasserstoff steht, mit Halogenierungsmitteln in Gegenwart von basischen Hilfsmitteln gemäß dem Reaktionsschema (IX) umgesetzt werden. 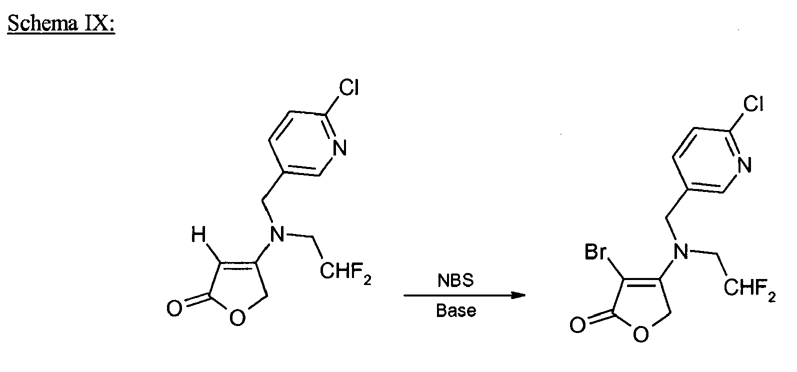
NBS: N-Brom-succinimid

In den als Ausgangsstoffe benötigten Verbindungen der Formel (I) haben A, B, R¹ und R³ die weiter oben genannte Bedeutung, der Substituent R² hat die Bedeutung von Wasserstoff.

Diese Verbindungen der Formel (I) können nach den weiter oben genannten Herstellverfahren 1 bis 3 erhalten werden.

Im Allgemeinen ist es vorteilhaft, die Halogenierung in Gegenwart von Verdünnungsmitteln durchzuführen. Verdünnungsmittel werden vorteilhaft in einer solchen Menge eingesetzt, dass das Reaktionsgemisch während des ganzen Verfahrens gut rührbar bleibt. Als Verdünnungsmittel zur Durchführung der Halogenierung kommen alle unter den Reaktionsbedingungen inerten organischen Lösungsmittel in Frage.

Als Halogenierungsmittel zur Durchführung des erfindungsgemäßen Verfahrens können alle geeigneten Halogenierungsmittel eingesetzt werden, beispielsweise *N-*Halogen-Verbindungen.

Beispielhaft seien genannt *N-*Halogenamine wie 1-Chlormethyl-4-fluordiazoniabicyclo[2.2.2]octan-bis-(tetrafluoroborat) (Selectfluor^{®}), *N,N-*Dihalogenamine, *N*-Halogen-carbonsäureamide, *N-*Halogen-carbamidsäureester, *N-*Halogen-harnstoff, *N-*Halogen-sulfonylamide, *N-*Halogendisulfonylamide, *N-*Halogen-sulfonylimide wie *N-*Fluor-bis[(trifluormethyl)sulfonyl]imid und *N-*Halogen-carbonsäurediamide wie *N*-Chlor-phthalimid, *N-*Brom-phthalimid, *N-*Iod-phthalimid, *N-*Chlor-succinimid (NCS), *N-*Brom-succinimid (NBS), *N-*Brom-saccharin oder *N-*Iod-succinimid.

Bevorzugte Halogenierungsmittel zur Durchführung der Halogenierung sind die *N-*Halogencarbonsäurediamide oder 1-Chlormethyl-4-fluordiazoniabicyclo[2.2.2]octan-bis-(tetrafluoroborat) (Selectfluor^{®}).

Bevorzugte Verdünnungsmittel zur Durchführung der Halogenierung sind Nitrile wie Acetonitril, Propionitril, Butyronitril, Isobutyronitril, Benzonitril, m-Chlorbenzonitril.

Man kann in das erfindungsgemäße Verfahren auch Gemische der genannten Lösungs- und Verdünnungsmittel einsetzen.

Besonders bevorzugte Verdünnungsmittel zur Durchführung des erfindungsgemäßen Verfahrens sind Nitrile wie Acetonitril, Propionitril oder Butyronitril.

Die Reaktionsdauer bei diesem Verfahren beträgt im Allgemeinen 10 Minuten bis 48 Stunden.

Die Umsetzung erfolgt bei Temperaturen zwischen -10°C und +100°C, bevorzugt zwischen 0°C und 60°C, besonders bevorzugt zwischen 10°C und Raumtemperatur.

Nach vollendeter Umsetzung wird der gesamte Reaktionsansatz eingeengt. Die nach Aufarbeitung anfallenden Produkte lassen sich in üblicher Weise durch Umkristallisieren, Vakuumdestillation oder Säulenchromatographie reinigen (vgl. auch die Herstellungsbeispiele).

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Allcylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie *N-*Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in rage:
   z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE-und/oder POP-Sorbitan- oder Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden. Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

Inhibitoren derNucleinsäure Synthese
   Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
Inhibitoren der Mitose und Zellteilung
   Benomyl, Carbendazim, Diethofencarb, Fuberidazole, Pencycuron, Thiabendazol, Thiophanatmethyl, Zoxamid
Inhibitoren der Atmungskette Komplex I
   Diflumetorim
Inhibitoren der Atmungskette Komplex II
   Boscalid, Carboxin, Fenfuram, Flutolanil, Furametpyr, Mepronil, Oxycarboxin, Penthiopyrad, Thifluzamid
Inhibitoren der Atmungskette Komplex III
   Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
Entkoppler
   Dinocap, Fluazinam
Inhibitoren der ATP Produktion
   Fentinacetat, Fentinchlorid, Fentinhydroxid, Silthiofam
Inhibitoren der Aminosäure- und Proteinbiosynthese
   Andoprim, Blasticidin-S, Cyprodinil, Kasugamycin, Kasugamycinhydrochlorid Hydrat, Mepanipyrim, Pyrimethanil
Inhibitoren der Signal-Transduktion
   Fenpiclonil, Fludioxonil, Quinoxyfen
Inhibitoren der Fett- und Membran Synthese
   Chlozolinat, Iprodion, Procymidon, Vinclozolin
   Ampropylfos, Kalium-Ampropylfos, Edifenphos, Iprobenfos (IBP), Isoprothiolan, Pyrazophos
   Tolclofos-methyl, Biphenyl
   Iodocarb, Propamocarb, Propamocarb hydrochlorid
Inhibitoren der Ergosterol Biosynthese
   Fenhexamid,
   Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol, Aldimorph, Dodemorph, Dodemorphacetat, Fenpropimorph, Tridemorph, Fenpropidin, Spiroxamin,
   Naftifin, Pyributicarb, Terbinafin
Inhibitoren der Zellwand Synthese
   Benthiavalicarb, Bialaphos, Dimethomorph, Flumorph, Iprovalicarb, Polyoxins, Polyoxorim, Validamycin A
Inhibitoren der Melanin Biosynthese
   Capropamid, Diclocymet, Fenoxanil, Phtalid, Pyroquilon, Tricyclazol
Resistenzinduktion
   Acibenzolar-S-methyl, Probenazol, Tiadinil
Multisite
   Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, I-minoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Unbekannter Mechanismus
   Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, *N-*(4-Chlor-2-nitrophenyl)-*N-*ethyl-4-methyl-benzenesulfonamid, 2-Amino-4-methyl-*N-*phenyl-5-thiazolecarboxamid, 2-Chlor-*N-*(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)-benzacetat, 4-Chlor-alpha-propinyloxy-*N* [2-[3-methoxy-4-(2-propinyloxy)phenyl]ethyl]-benzacetamide, (2S)-*N*-[2-[4-[[3-(4-chlorophenyl)-2-propinyl]oxy]-3-methoxyphenyl]ethyl]-3-methyl-2-[(methylsulfonyl)amino]-butanamid, 5-Chlor-7-(4-methylpiperidin-1-yl)-6-(2,4,6-trifluorophenyl)[1,2,4]triazolo[1,5-a]pyrimidin, 5-Chlor-6-(2,4,6-trifluorophenyl)-*N*-[(1R)-1,2,2-trimethylpropyl][1,2,4]triazolo[l,5-a]pyrimidin-7-amin, 5-Chlor-*N*-[(1R)-1,2-dimethylpropyl]-6-(2,4,6-trifluorophenyl) [1,2,4]triazolo[1,5-a]pyrimidin-7-amine, *N*-[1-(5-Brom-3-chloropyridin-2-yl)ethyl]-2,4-dichloronicotinamid, *N*-(5-Brom-3-chlorpyridin-2-yl)methyl-2,4-dichlornicotinamid, 2-Butoxy-6-iod-3-propyl-benzopyranon-4-on, *N*-{(Z)-[(cyclopropylmethoxy) imino] [6-(difluormethoxy)-2,3-difluorphenyl]methyl}-2-benzacetamid, *N*-(3- Ethyl- 3,5,5-trimethyl-cyclohexyl)-3-formylamino-2-hydroxy-benzamid , 2-[[[[1-[3(1Fluor-2-phenylethyl)oxy]phenyl]ethyliden]amino]oxy]methyl]-alpha-(methoxyimino)-*N*-methyl-alphaE-benzacetamid, *N*-{2-[3-Chlor-5-(trifluormethyl)pyridin-2-yl]ethyl}-2-(trifluoromethyl)benzamid, *N*-(3',4'-dichlor-5-fluorbiphenyl-2-yl)-3-(difluormethyl)-1-methyl-1H-pyrazol-4-carboxamid, *N*-(6-Methoxy-3-pyridinyl)-cyclopropan carboxamid, 1-[(4-Methoxyphenoxy)methyl]-2,2-dimethylpropyl-1H-imidazol-1- carbonsäur e , O-[1-[(4-Methoxyphenoxy)methyl]-2,2-dimethylpropyl]-1H-imidazol- 1- carbothioic acid, 2-(2-{[6-(3-Chlor-2-methylphenoxy)-5-fluorpyrimidin-4-yl]oxy}phenyl)-2-(methoxyimino)-*N-*methylacetamid

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
Carbamate,
   zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
Organophosphate,
   zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/- ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
   Pyrethroide,
   zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
   DDT
   Oxadiazine,
   zum Beispiel Indoxacarb
   Semicarbazon,
   zum Beispiel Metaflumizon (BAS3201)
Acetylcholin-Rezeptor-Agonisten/-Antagonisten
   Chloronicotinyle,
   zum Beispiel Acetamiprid, Clothianidin, Dinotefuran, Imidacloprid, Nitenpyram, Nithiazine, Thiacloprid, Imidaclothiz, AKD-1022, Thiamethoxam
   Nicotine, Bensultap, Cartap
Acetylcholin-Rezeptor-Modulatoren
   Spinosyne,
   zum Beispiel Spinosad, Spinetoram (XDE-175)
GABA-gesteuerte Chlorid-Kanal-Antagonisten
   Organochlorine,
   zum Beispiel Camphechlor, Chlordane, Endosulfan, Gamma-HCH, HCH, Heptachlor, Lindane, Methoxychlor
   Fiprole,
   zum Beispiel Acetoprole, Ethiprole, Fipronil, Pyrafluprole, Pyriprole, Vaniliprole
Chlorid-Kanal-Aktivatoren
   Mectine,
   zum Beispiel Abamectin, Emamectin, Emamectin-benzoate, Ivermectin, Lepimectin, Milbemycin
Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
   Diacylhydrazine,
   zum Beispiel Chromafenozide, Halofenozide, Methoxyfenozide, Tebufenozide
Inhibitoren der Chitinbiosynthese
   Benzoylharnstoffe,
   zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
   zum Beispiel Azocyclotin, Cyhexatin, Fenbutatin-oxide
Entkoppler der oxidativen Phoshorylierung durch Unterbrechung des H-Protongradienten
   Pyrrole,
   zum Beispiel Chlorfenapyr
   Dinitrophenole,
   zum Beispiel Binapacyrl, Dinobuton, Dinocap, DNOC
Seite-I-Elektronentransportinhibitoren
   METI's,
   zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
   Rotenone
Seite-III-Elektronentransportinhibitoren
   Acequinocyl, Fluacrypyrim
Mikrobielle Disruptoren der Insektendarmmembran
   Bacillus thuringiensis-Stämme
Inhibitoren der Fettsynthese
   Tetronsäuren,
   zum Beispiel Spirodiclofen, Spiromesifen
   Tetramsäuren,
   zum Beispiel Spirotetramat
   Carboxamide,
   zum Beispiel Flonicamid
   Oktopaminerge Agonisten,
   zum Beispiel Amitraz
Inhibitoren der Magnesium-stimulierten ATPase,
   Propargite
   Nereistoxin-Analoge,
   zum Beispiel Thiocyclam hydrogen oxalate, Thiosultap-sodium
Agonisten des Ryanodin-Rezeptors,
   Benzoesäuredicarboxamide,
   zum Beispiel Flubendiamid
   Anthranilamide,
   zum Beispiel Rynaxypyr (3-Bromo-*N*-{4-chloro-2-methyl-6-[(methylamino)carbonyl]-phenyl}-1-(3-chloropyridin-2-yl)-1 H-pyrazole-5-carboxamide)
Biologika, Hormone oder Pheromone
   Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
   Begasungsmittel,
   zum Beispiel Aluminium phosphide, Methyl bromide, Sulfuryl fluoride
   Fraßhemmer,
   zum Beispiel Cryolite, Flonicamid, Pymetrozine
   Milbenwachstumsinhibitoren,
   zum Beispiel Clofentezine, Etoxazole, Hexythiazox
   Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt und dessen Genprodukt Wirksamkeit gegen Maiszünsler und/oder Maiswurzel-Bohrer zeigt. Besonders bevorzugt handelt es sich dabei um ein heterologes Gen, das aus Bacillus thuringiensis stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und Cry-IF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizidtolerante Pflanzen seien Maissorten, Baumwollsorten und Sojasorten genannt, die unter den Handelsbezeichnungen Roundup Ready@ (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufinilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum,
Ptilinus pecticornis, Dendrobium pertinex, Ernobius mollis, Priobium carpini, Lyctus brunneus,
Lyctus africanus, Lyctus planicollis, Lyctus linearis, Lyctus pubescens, Trogoxylon aequale,
Minthes rugicollis, Xyleborus spec. Tryptodendron spec. Apate monachus, Bostrychus capucins,
Heterobostrychus brunneus, Sinoxylon spec. Dinoderus minutus;

Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.
Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele:

### Verfahren 1

### Variante A

### 4-[[(6-Chlorpyridin-3-yl)methyl](2,2-difluorethyl)amino]furan-2(5H)-on

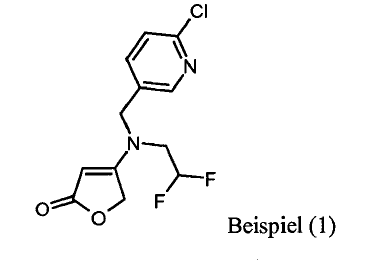

21.90 g (106.0 mmol) N-[(6-Chlorpyridin-3-yl)methyl]-2,2-difluorethan-1-amin (III-1), 14.85 g (148.4 mmol) Tetronsäure und 183 mg (1.1 mmol) 4-Toluolsulfonsäure werden in 250 mL Toluol 2 Stunden am Wasserabscheider unter Rückfluss erhitzt. Nach Einengen der Reaktionsmischung im Vakuum nimmt man mit Essigsäureethylester auf, wäscht nacheinander zweimal mit 1 N wässriger Salzsäure, zweimal mit 1 N wässriger Natriumhydroxid-Lösung und einmal mit gesättigter Natriumchlorid-Lösung. Man trocknet die organischen Phase über Natriumsulfat und engt sie im Vakuum ein. Reinigung des Rückstandes durch Säulenchromatographie über Kieselgel (Kieselgel 60 - Merck, Korngröße: 0.04 bis 0.063 mm) mit dem Laufmittel Essigsäureethylester liefert 15.9 g (52 % der Theorie) 4-[[(6-Chlor-pyridin-3-yl)methyl](2,2-difluorethyl)amino]furan-2(5H)-on.
¹H-NMR (CD₃CN, δ, ppm) = 3.59 (td, 2 H), 4.51 (s, 2 H), 4.76 (s, 1 H), 4.80 (s, 2 H), 6.03 (tt, 1 H), 7.38 (d, 1 H), 7.64 (dd, 1 H), 8.28 (d, 1 H).

### Variante B

### 4-[[(6-Chlorpyridin-3-yl)methyl](3-fluor-n-propyl)amino]furan-2(5H)-on

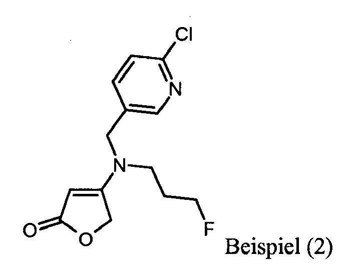

0.52 g (5,18 mmol) Tetronsäure wird in 1.13 ml Essigsäure vorgelegt und bei Raumtemperatur langsam mit 1.00 g (4.93 mmol) N [(6-Chlorpyridin-3-yl)methyl]-3-fluor-propan-1-amin (III-2) versetzt. Anschließend wird das gesamte Reaktionsgemisch ca. 18 Stunden bei Raumtemperatur weitergerührt. Nach Einengen der Reaktionsmischung im Vakuum nimmt man mit Dichlormethan auf und wäscht mit Wasser. Nach Phasentrennung wird die wässrige Phase zweimal mit Dichlormethan extrahiert. Danach werden die vereinigten organischen Phasen mit, 1 N Natriumhydroxid-Lösung alkalisch (pH > 9) gestellt und die Phasen getrennt. Die wässrige Phase wird zweimal mit Dichlormethan extrahiert und nach Phasentrennung werden die vereinigten organischen Phasen über Natriumsulfat getrocknet. Nach Einengen der organischen Phase im Vakuum und Reinigung des Rückstandes durch Säulenchromatographie über Kieselgel (Kieselgel 60 - Merck, Korngröße: 0.04 bis 0.063 mm) mit dem Laufmittelgemisch Essigsäureethylester : Cyclohexan (9:1) erhält man 191 mg (14 % der Theorie) 4-[[(6-Chlor-pyridin-3-yl)methyl](3-fluor-n-propyl)amino]furan-2(5H)-on.
¹H-NMR (CD₃CN, δ, ppm) = 1.95 (m, 2H), 3.30 (t, 2H), 4.40 (s, 2H), 4.45 (dt, 2H), 4.65 (s, 1H), 4.80 (s, 2H), 7.40 (d, 1H), 7.65 (dd, 1H), 8.28 (d, 1H).

Analog zu dieser Vorschrift wurden auch die Verbindungen (16) und (17) hergestellt.

### Verfahren 2

### 4- [[(6-Chlorpyridin-3-yl)methyl] (3,3-dichlorprop-2-en-1-yl)amino]furan-2(5H)-on

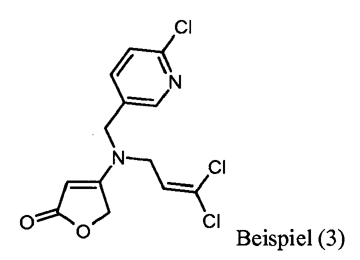

350 mg (1.56 mmol) 4-[[(6-Chlorpyridin-3-yl)methyl]amino]furan-2(5H)-on (Ia-1; vgl. EP 0539588 Al) und 124 mg (3.12 mmol) einer 60%igen Dispersion von Natriumhydrid in Mineralöl werden 3 h in 100 mL Tetrahydrofuran unter Rückfluss erhitzt. Nach Abkühlen auf Raumtemperatur gibt man 592 mg (3.12 mmol) 3-Brom-1,1-dichlorprop-1-en (vgl. WO 8800183 A1) hinzu und erhitzt weitere 5 h unter Rückfluss. Nach Abkühlen der Reaktionsmischung auf Raumtemperatur und Zugabe von Methanol engt man im Vakuum ein. Man nimmt den Rückstand mit Essigsäureethylester auf und wäscht nacheinander zweimal mit 1 N wässriger Salzsäure, zweimal mit 1 N wässriger Natriumhydroxid-Lösung und einmal mit gesättigter Natriumchlorid-Lösung. Man trocknet anschließend die organischen Phase über Natriumsulfat und engt sie im Vakuum ein. Durch Reinigung des Rückstandes mittels Säulenchromatographie über Kieselgel (Kieselgel 60 - Merck, Korngröße: 0.04 bis 0.063 mm) mit dem Laufmittelgemisch Essigsäureethylester : Cyclohexan (2:1) erhält man 264 mg (50 % der Theorie) 4- [[(6-Chlorpyridin-3 -yl)methyl] (3,3 -dichlorprop-2-en-1 -yl)amino] furan-2(5H)-on.
¹H-NMR (CD₃CN, δ ppm) = 3.90 (d, 2 H), 4.38 (s, 2 H), 4.71 (s, 1 H), 4.80 (s, 2 H), 6.02 (t, 1 H), 7.38 (d, 1 H), 7.66 (dd, 1 H), 8.29 (d, 1 H).

Analog zu dieser Vorschrift wurden auch die Verbindungen (9) und (12) hergestellt.

### Verfahren 3

### 4-[[(6-Chlorpyridin-3-yl)methyl](2-fluorethyl)amino]furan-2(5H)-on

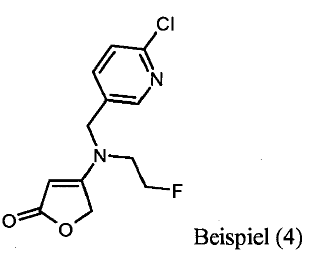

1.00 g (6.89 mmol) 4-[(2-Fluorethyl)amino]furan-2(5H)-on (VI-1) und 0.55 g (13.78 mmol) einer 60%igen Dispersion von Natriumhydrid in Mineralöl werden 2 h in 200 ml Tetrahydrofuran unter Rückfluss erhitzt. Nach Abkühlen auf Raumtemperatur gibt man 2.23 g (13.78 mmol) 2-Chlor-5-chlormethyl-pyridin hinzu und erhitzt weitere 4 Stunden unter Rückfluss. Man kühlt die Reaktionsmischung auf Raumtemperatur ab und gibt Methanol hinzu. Nach Einengen der Reaktionsmischung im Vakuum nimmt man mit Essigsäureethylester auf, wäscht nacheinander zweimal mit 1 N wässriger Salzsäure, zweimal mit 1 N wässriger Natriumhydroxid-Lösung und einmal mit gesättigter Natriumchlorid-Lösung. Man trocknet anschließend die organischen Phase über Natriumsulfat und engt sie im Vakuum ein. Durch Reinigung des Rückstandes mittels Säulenchromatographie über Kieselgel (Kieselgel 60 - Merck, Korngröße: 0.04 bis 0.063 mm) mit dem Laufmittelgemisch Essigsäureethylester : Cyclohexan (9:1) erhält man 949 mg (50 % der Theorie) 4-[[(6-Chlorpyridin-3-yl)methyl](2-fluorethyl) amino]furan-2(5H)-on.
¹H-NMR (CD₃CN, δ, ppm) = 3.50 (dt, 2 H), 4.50 (s, 2 H), 4.57 (dt, 2 H), 4.65 (s, 1 H), 4.79 (s, 2 H), 7.38 (d, 1H), 7.65 (dd, 1 H), 8.28 (d, 1 H).

Analog zu dieser Vorschrift wurden auch die Verbindungen (10), (11), (13), (14) und (15) hergestellt.

### (E/Z)-4-[[(6-Chlorpyridin-3-yl)methyl](2-fluor-vinyl)amino]-5-methylfuran-2(5H)-on

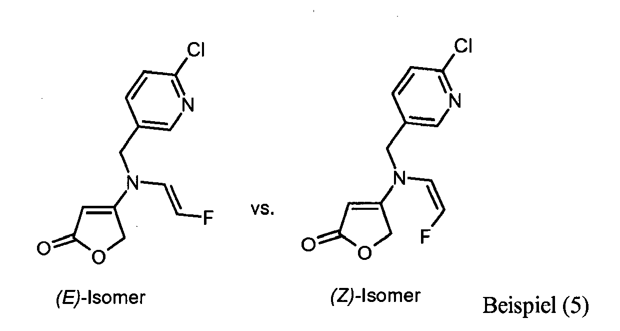

68 mg (0.2 2 mmol) 4-[[(6-Chlorpyridin-3-yl)methyl](2-chlor-2-fluorethyl)amino]furan-2(5H)-on (17) werden in 5 mL Ethanol vorgelegt, mit 38 mg (0.67 mmol) Kaliumhydroxid versetzt und das Reaktionsgemisch wird ca. 18 Stunden bei Raumtemperatur gerührt. Danach wird der Reaktionsansatz zwischen Essigsäureethylester und Wasser verteilt und die organische Phase abgetrennt. Die organische Phase wird mit gesättigter Natriumchlorid-Lösung gewaschen, über Natriumsulfat getrocknet und im Vakuum eingeengt. Durch Reinigung des Rückstandes mittels Säulenchromatographie über Kieselgel (Kieselgel 60 - Merck, Korngröße: 0.04 bis 0.063 mm) mit dem Laufmittelgemisch Essigsäureethylester : Cyclohexan (2:1) erhält man 57 mg (94 % der Theorie) 4-[[(6-Chlorpyridin-3-yl)methyl](2-fluor-vinyl)amino]-5-methylfuran-2(5H)-on als *(E*/*Z)*-Isomerengemisch (15:85).
¹H-NMR (CD₃CN, δ, ppm) = 4.75 (s, 2H), 4.84 (s, 2H), 4.88 (s, 1H), 5.60 (dd, 1H), 6.37 (dd, 1H), 7.39 (d, 1H), 7.67 (dd, 1H), 8.29 (d, 1H) [*(Z)*-Isomer; 85 % lt. NMR].
¹H-NMR (CD₃CN, δ, ppm) = 6.90 (dd, 1H) [*(E)*-Isomer; 15 % lt. NMR].

### 4-[[(6-Chlorpyridin-3-yl)methyl](2,2-difluorethyl)amino]-5-methylfuran-2(5H)-on

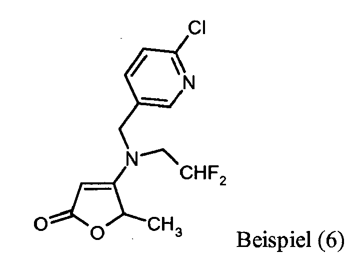

300 mg (1.04 mmol) 4-[[(6-Chlorpyridin-3-yl)methyl](2,2-difluorethyl)amino]furan-2(5H)-on (1) werden in 10 mL Tetrahydrofuran gelöst, auf -78°C abgekühlt und mit 611 µL (1.04 mmol) einer 1.7 M Lösung von *tert*.-Butyllithium in Pentan versetzt. Nach 30 min Rühren bei -78°C gibt man 65 µL (1.04 mmol) Methyliodid hinzu, rührt weitere 30 min bei -78°C und wärmt auf Raumtemperatur auf. Einengen im Vakuum und Reinigung des Rückstandes durch Säulenchromatographie über Kieselgel (Kieselgel 60 - Merck, Korngröße: 0.04 bis 0.063 mm) mit dem Laufmittelgemisch Essigsäureethylester : Cyclohexan (2:1) liefern 152 mg (47 % der Theorie) 4-[[(6-Chlorpyridin-3-yl)methyl] (2,2-difluorethyl)amino]-5-methylfuran-2(5H)-on.
¹H-NMR (CD₃CN, δ ppm) = 1.48 (d, 3 H), 3.59 (m, 2 H), 4.48 (d, 1 H), 4.58 (d, 1 H), 4.73 (s, 1 H), 5.10 (q, 1 H), 6.06 (tt, 1 H), 7.40 (d, 1 H), 7.65 (dd, 1 H), 8.28 (d, 1 H).

### 3-Brom-4-[[(6-chlorpyridin-3- yl)methyl](2,2-difluorethyl)amino ]furan-2(5H)-on

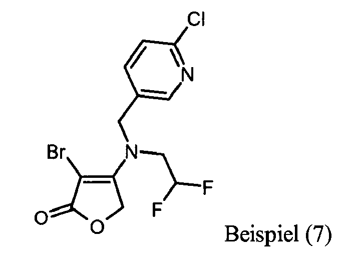

313 mg (1.0 8 mmol) 4-[[(6-Chlorpyridin-3-yl)methyl](2,2-difluorethyl)amino]furan-2(5H)-on (1) werden in 20 mL Acetonitril gelöst und bei Raumtemperatur mit 166 µL (1.19 mmol) Triethylamin und 386 mg 2.17 mmol) *N* Bromsuccinimid versetzt. Nach 3 Stunden Rühren wird im Vakuum eingeengt. Reinigung des Rückstandes durch Säulenchromatographie über Kieselgel (Kieselgel 60 - Merck, Korngröße: 0.04 bis 0.063 mm) mit dem Laufmittelgemisch Essigsäureethylester : Cyclohexan (2:1) liefert 312 mg (63 % der Theorie) 3-Brom-4-[[(6-chlorpyridin-3-yl)methyl](2,2-difluorethyl)amino] furan-2(5H)-on.
¹H-NMR (CD₃CN, δ, ppm) = 3.80 (td, 2 H), 4.83 (s, 2 H), 4.85 (s, 2 H), 6.08 (tt, 1 H), 7.40 (d, 1 H), 7.66 (dd, 1 H), 8.30 (d, 1 H).

### 3-Chlor-4-[[(6-chlorpyridin-3-yl)methyl](2,2-difluorethyl)amino]furan-2(5H)-on

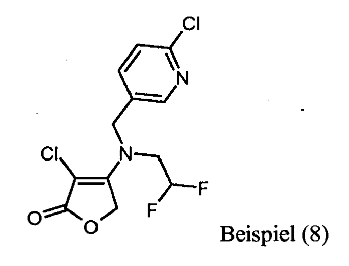

Die Halogenierungsreaktion erfolgt analog der Reaktionsvorschrift des Beispiels 6 unter Verwendung von:
329 mg (1.14 mmol) 4-[[(6-Chlorpyridin-3-yl)methyl](2,2-difluorethyl)amino]furan-2(5H)-on (1)
0.17 ml (1.25 mmol) Triethylamin
304 mg (2.28 mmol) *N* Chlor-succinimid
20 mL Acetonitril

Der zurückbleibende Rückstand wird durch Säulenchromatographie über Kieselgel (Kieselgel 60 - Merck, Korngröße: 0.04 bis 0.063 mm) mit dem Laufmittelgemisch Essigsäureethylester : Cyclohexan (2:1) gereinigt. Man erhält 292 mg (63 % der Theorie) 3-Chlor-4-[[(6-chlorpyridin-3-yl)methyl] (2,2-difluorethyl)amino] furan-2(5H)-on.
¹H-NMR (CD₃CN, δ ppm) = 3.79 (td, 2H), 4.78 (s, 2H), 4.82 (s, 2H), 6.07 (tt, 1H), 7.40 (d, 1H), 7.67 (dd, 1H), 8.30 (d, 1H).

In der nachstehenden Tabelle 1 sind weitere Verbindungen (9) bis (17) der Formel (I) aufgeführt.

**Tabelle 1**

| Verbindungen der Formel | | | | | | |
|---|---|---|---|---|---|---|
| 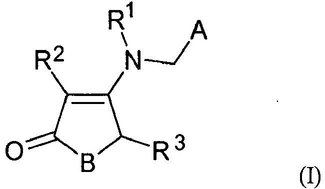 | | | | | | |

| **Bsp.-Nr.** | **A** | **B** | **R¹** | **R²** | **R³** | **Physikalische Daten: ¹H-NMR, δ [ppm]** |
|---|---|---|---|---|---|---|
| 9 | 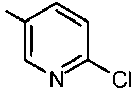 | O | 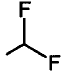 | H | H | CD₃Cl, δ = 4.62 (s, 2H), 4.93 (s, 2H), 5.00 (s, 1H), 6.50 (t, 1H), 7.38 (d, 1H), 7.57 (dd, 1H), 8.35 (d, 1H) |
| 10 | 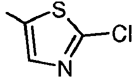 | O | 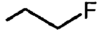 | H | H | CD₃CN, δ = 3.48 (dt, 2H), 4.57 (dt, 2H), 4.58 (s, 2H), 4.71 (s, 1H), 4.78 (s, 2H), 7.50 (s, 1H) |
| 11 | 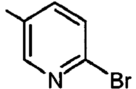 | O | 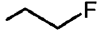 | H | H | CD₃CN, δ = 3.50 (dt, 2H), 4.47 (s, 2H), 4.55 (dt, 2H), 4.65 (s, 1H), 4.80 (s, 2H), 7.53 (s, 2H), 8.28 (s, 1H) |
| 12 | 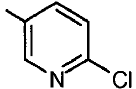 | O | 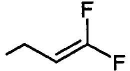 | H | H | CD₃CN, δ = 3.78 (dm, 2H), 4.39 (s, 2H), 4.50 (dtd, 1H), 4.68 (s, 1H), 4.80 (s, 2H), 7.38 (d, 1H), 7.65 (dd, 1H), 8.28 (d, 1H) |
| 13 | 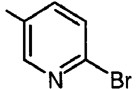 | O | 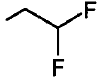 | H | H | CD₃CN, δ = 3.58 (td, 2H), 4.50 (s, 2H), 4.75 (s, 1H), 4.80 (s, 2H), 6.05 (tt, 1H), 7.55 (s, 2H), 8.28 (s, 1H) |
| 14 | 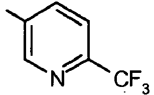 | O | 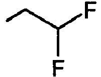 | H | H | CD₃CN, δ = 3.61 (td, 2H), 4.63 (s, 2H), 4.75 (s, 1H), 4.80 (s, 2H), 6.05 (tt, 1H), 7.75 (d, 1H), 7.85 (d, 1 H), 8.62 (s, 1H) |
| 15 | 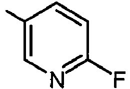 | O | 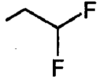 | H | H | CD₃CN, δ = 3.58 (td, 2H), 4.52 (s, 2H), 4.76 (s, 1H), 4.81 (s, 2H), 6.03 (tt, 1H), 7.00 (dd, 1H), 7.78 (td, 1H), 8.10 (s, 1H) |
| 16 | 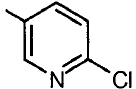 | CH₂ | 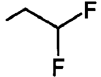 | H | H | CD₃CN, δ = 2.30 (m, 2H), 2.74 (m, 2H), 3.71 (td, 2H), 4.61 (s, 2H), 5.01 (s, 1H), 6.05 (tt, 1H), 7.40 (d, 1H), 7.62 (dd, 1H), 8.28 (d, 1H) |
| 17 | 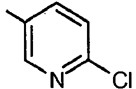 | O | 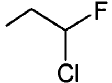 | H | H | CD₃CN, δ = 3.75 (m, 2H), 4.55 (s, 2H), 4.75 (s, 1H), 4.82 (s, 2H), 6.43 (ddd, 1H), 7.38 (d, 1H), 7.65 (dd, 1H), 8.28 (d, 1H) |

### Herstellung von Ausgangsverbindungen

### Verbindungen der Formel (la)

### Ia-1

**4-[[(6-Chlorpyridin-3-yl)methyl]amino]furan-2(5H)-on (vgl.** EP 0539588A1**)**

5.00 g (35.1 mmol) 1-(6-Chlorpyridin-3-yl)methylamin, 3.51 g (35.1 mmol) Tetronsäure und 20 mg (0.12 mmol) 4-Toluolsulfonsäure werden in 200 mL Toluol 24 Stunden am Wasserabscheider unter Rückfluss erhitzt. Nach Einengen der Reaktionsmischung wird der verbleibende Rückstand durch Säulenchromatographie über Kieselgel (Kieselgel 60 - Merck, Korngröße: 0.04 bis 0.063 mm) mit dem Laufmittel Essigsäureethylester gereinigt. Man erhält man 4.96 g (63 % der Theorie) 4-[[(6-Chlorpyridin-3-yl)methyl]amino]furan-2(5H)-on, das für Folgereaktionen verwendet werden kann.
¹H-NMR (CDC1₃, δ ppm) = 4.35 (d, 2 H), 4.70 (s, 2 H), 4.80 (s, 1 H), 4.95 (br. s, 1 H), 7.36 (d, 1H), 7.61 (dd, 1 H), 8.37 (d, 1 H).

### Verbindungen der Formel (III)

### III-1

### N-[( 6-Chlorpyridin-3-yl)methyl]-2,2-difluorethan-1-amin

41,57 g (256.6 mmol) 2-Chlor-5-chlormethyl-pyridin, 20.80 g (256.6 mmol) 2,2-Difluorethan-l-amin und 35.8 mL (256.6 mmol) Triethylamin werden in 500 mL Acetonitril 21 Stunden bei 45°C gerührt. Nach Einengen der Reaktionsmischung im Vakuum nimmt man mit 1 N wässriger Salzsäure auf und wäscht mit Essigsäureethylester. Die wässrige Phase wird mit 2.5 N wässriger Natriumhydroxid-Lösung alkalisch gestellt und mit Essigsäureethylester mehrmals extrahiert. Einengen der organischen Phase im Vakuum liefert 28.6 g (53 % der Theorie) *N* [(6-Chlorpyridin-3-yl)methyl]-2,2-difluorethan-1-amin.
¹H-NMR (CD₃CN, δ, ppm) = 2.93 (td, 2 H), 3.80 (s, 2 H), 5.85 (tt, 1 H), 7.33 (d, 1 H), 7.71 (dd, 1H), 8.30 (d, 1 H).

### In analoger Weise kann dargestellt werden:

### III-2

### N-[(6-Chlorpyridin-3-yl)methyl]-3-fluor-propan-1-amin

LCMS *(mlz,* %) = 203 (MH⁺, 100).

### III-3

### N [(6-Chlorpyridin-3-yl)methyl]-2-chlor-2-fluorethan-1-amin

LCMS *(m*/*z,* %) = 223 (MH⁺, 100).

### Verbindungen der Formel (V)

### Va-1

### 2-Chlor-fluorethan-1-amin:

a) 5.00 g (44.8 mmol) 2-Chlor-2-fluoracetamid werden in 50.0 ml Tetrahydrofuran verrührt und tropfenweise mit 51.6 ml (103.1 mmol) Boran-Dimethylsulfid-Komplex versetzt. Anschließend wird das Reaktionsgemisch eine Stunde bei Rückflusstemperatur gerührt. Nach dem Abkühlen werden vorsichtig 155.7 ml (0.45 mmol) 10%ige Chlorwasserstoffsäure zugegeben und eine weitere Stunde bei Rückflusstemperatur gerührt. Danach wird das Tetrahydrofuran im Vakuum entfernt, der Rückstand abgekühlt (Eisbad), mit Diethylether verrührt und die Mischung mit Natriumhydroxid-Lösung auf pH 9 eingestellt. Man extrahiert dreimal mit Diethylether und versetzt die vereinigte organische Phase zur Hydrochloridbildung mit 44.8 ml (89.7 mmol) Chlorwasserstoff in Dioxan. Das ausgefallene Hydrochlorid wird abgetrennt und nochmals nachgewaschen. Man erhält 3,48 g (58 % der Theorie) 2-Chlor-3-fluorethan-1-amin Hydrochlorid.
b) Zur Bildung der freien Base werden 3.40 g (25.4 mmol) 2-Chlor-3-fluorethan-1-amin Hydrochlorid (zuvor im Hochvakuum getrocknet) mit 2.03 g (50.8 mmol) Natrumhydroxidpulver versetzt. Anschließend kann die freie Base bei 100 °C abdestilliert werden. Man erhält 2.10 g (85 % der Theorie) 2-Chlor-3-fluorethan-1-amin, das zur Synthese des *N* [(6-Chlorpyridin-3-yl)methyl]-2-chlor-2-fluorethan-1-amins (III-3) verwendet werden kann.
¹H-NMR (CD₃CN, δ ppm) = 3.07 (dm, 2 H), 6.17 (dt, 1 H).

### Verbindungen der Formel (VI)

### VI-1

### 4-[(2-Fluorethyl)amino]furan-2(5H)-on

550 mg (4.97 mmol) 2-Fluorethylaminhydrochlorid, 547 mg (5.47 mmol) Tetronsäure, 408 mg (4.97 mmol) Natriumacetat und 9 mg (0,05 mmol) 4-Toluolsulfonsäure werden in 50 mL Toluol 5 Stunden am Wasserabscheider unter Rückfluss erhitzt. Nach Einengen der Reaktionsmischung im Vakuum nimmt man mit Essigsäureethylester auf und wäscht mit 1 N wässriger Natriumhydroxid-Lösung. Die wässrige Phase wird mehrmals mit Essigsäureethylester extrahiert und die vereinigten organischen Phase über Natriumsulfat getrocknet. Nach Einengen der organischen Phase im Vakuum und Reinigung des Rückstandes durch Umkristallisation aus Essigsäureethylester/Cyclohexan erhält man 300 mg (42 % der Theorie) 4-[(2-Fluorethyl)amino]furan-2(5H)-on.
¹H-NMR (CD₃CN, δ, ppm) = 3.40 (dq, 2 H), 4.52 (dt, 2H), 4.61 (s, 1 H), 4.62 (s, 2 H), 5.80 (br. s, 1 H).

In analoger Weise kann dargestellt werden:

### VI-2

### 4-[(2,2-Difluorethyl)amino ]furan-2(5H)-on

¹H-NMR (CD₃CN, δ, ppm) = 3.50 (tm, 2 H), 4.65 (s, 2 H), 4.71 (s, 1 H), 5.78 (br.s, 1 H), 5.98 (tt, 1 H).

### Biologische Beispiele

### Beispiel Nr. 1

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: siehe Tabelle

| Beispiel | Wirkstoffkonzentration in g/ha | Abtötungsgrad in % nach 5 Tagen |
|---|---|---|
| Beispiel 3 | 500 | 100 |
| Beispiel 12 | 500 | 100 |
| Beispiel 10 | 500 | 100 |
| Beispiel 11 | 500 | 100 |
| Beispiel 14 | 500 | 100 |
| Beispiel 13 | 500 | 100 |
| Beispiel 15 | 500 | 100 |
| Beispiel 2 | 500 | 100 |
| Beispiel 7 | 500 | 100 |
| Beispiel 8 | 500 | 100 |
| Beispiel 6 | 500 | 100 |
| Beispiel 17 | 500 | 100 |
| Beispiel 5 | 500 | 100 |

### Beispiel Nr. 2

### Myzus-Test; oral; (MYZUPE O)

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit allen Stadien der Grünen Pfirsichblattlaus *(Myzus persicae)* besetzt, durch Saugen an der Wirkstoffzubereitung der gewünschten Konzentration wird behandelt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: siehe Tabelle

| Beispiel | Wirkstoffkonzentration in ppm | Abtötungsgrad in % nach 5 Tagen |
|---|---|---|
| Beispiel 2 | 20 | 100 |

### Beispiel Nr. 3

### Nilaparvata lugens-Test (NILALU hydroponische Behandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Die Wirkstoffzubereitung wird in Wasser pipettiert. Die angegebene Konzentration bezieht sich auf die Wirkstoffmenge pro Volumeneinheit Wasser (mg/l = ppm), anschließend wird mit der Braunrückigen Reiszikade *(Nilaparvata lugens)* infiziert.

Nach der gewünschten Zeit wird die Wirkung in % bestimmt. Dabei bedeutet 100 %, dass alle Reiszikaden abgetötet wurden; 0 % bedeutet, dass keine Reiszikaden abgetötet wurden.

Bei diesem Test zeigen z. B. die folgenden Verbindungen der Herstellungsbeispiele gute Wirksamkeit: siehe Tabelle

| Beispiel | Wirkstoffkonzentration in g/ha | Abtötungsgrad in % nach 7 Tagen |
|---|---|---|
| Beispiel 10 | 100 | 100 |
| Beispiel 13 | 100 | 100 |

### Beispiel Nr. 4

### Phaedon-Test (PHAECO Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit: siehe Tabelle

| Beispiel | Wirkstoffkonzentration in g/ha | Abtötungsgrad in % nach 7 Tagen |
|---|---|---|
| Beispiel 11 | 500 | 100 |
| Beispiel 13 | 500 | 100 |
| Beispiel 15 | 500 | 100 |
| Beispiel 17 | 500 | 100 |

### Beispiel Nr. 5

### Meloidogyne-Test (MELGIN Spritzbehandlung)

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, Meloidogyne incognita-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele gute Wirksamkeit: siehe Tabelle

| Beispiel | Wirkstoffkonzentration in ppm | Abtötungsgrad in % nach 14 Tagen |
|---|---|---|
| Beispiel 10 | 20 | 80 |
| Beispiel 11 | 20 | 100 |

### Beispiel Nr. 6

### Myzus persicae -Test, hydroponische Behandlung (MYZUPE sys.)

| | | |
|---|---|---|
| Lösungsmittel: | 7 | Gewichtsteile Dimethylformamid |
| | | |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Wasser gemischt. Die angegebene Konzentration bezieht sich auf die Wirkstoffmenge pro Volumeneinheit Wasser (mg/1= ppm). Man füllt das behandelte Wasser in Gefäße mit einer Erbsenpflanze *(Pisum sativum),* anschließend wird mit der Grünen Pfirsichblattlaus *(Myzus persicae)* infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindungen der Herstellungsbeispiele gute Wirksamkeit: siehe Tabelle

| Beispiel | Wirkstoffkonzentration in ppm | Abtötungsgrad in % nach 6 Tagen |
|---|---|---|
| Beispiel 10 | 20 | 100 |
| Beispiel 11 | 20 | 100 |
| Beispiel 14 | 20 | 100 |
| Beispiel 13 | 20 | 100 |
| Beispiel 15 | 4 | 100 |
| Beispiel 8 | 4 | 98 |

### Beispiel Nr. 7

### Aphis gossypii -Test (APHIGO)

| | | |
|---|---|---|
| Lösungsmittel: | 7 | Gewichtsteile Dimethylformamid |
| | | |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter *(Gossypium hirsutum),* die stark von der Baumwollblattlaus *(Aphis gossypii)* befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindungen der Herstellungsbeispiele gute Wirksamkeit: siehe Tabelle

| Beispiel | Wirkstoffkonzentration in ppm | Abtötungsgrad in % nach 6 Tagen |
|---|---|---|
| Beispiel 10 | 100 | 98 |
| Beispiel 11 | 100 | 100 |
| Beispiel 14 | 100 | 100 |
| Beispiel 13 | 100 | 100 |
| Beispiel 15 | 100 | 100 |
| Beispiel 8 | 20 | 100 |

### Beispiel Nr.8

### Aphis gossypii-Test; (APHIGO G)

| | | |
|---|---|---|
| Lösungsmittel: | 7 | Gewichtsteile Dimethylformamid |
| | | |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Baumwollpflanzen *(Gossypium hirsutum),* die stark von der Baumwollblattlaus *(Aphis gossypii)* befallen sind, werden mit einer Wirkstoffzubereitung der gewünschten Konzentration angegossen.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: siehe Tabelle

| Beispiel | Wirkstoffkonzentration in ppm | Abtötungsgrad in % nach 10 Tagen |
|---|---|---|
| Beispiel 10 | 4 | 100 |

### Beispiel Nr. 9

### Myzus persicae -Test; (MYZUPE G)

| | | |
|---|---|---|
| Lösungsmittel: | 7 | Gewichtsteile Dimethylformamid |
| | | |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlpflanzen *(Brassica oleracea),* die stark von der Grünen Pfirsichblattlaus *(Myzus persicae)* befallen sind, werden mit einer Wirkstoffzubereitung der gewünschten Konzentration angegossen. Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit: siehe Tabelle.

| Beispiel | Wirkstoffkonzentration in ppm | Abtötungsgrad in % nach 10 Tagen |
|---|---|---|
| Beispiel 10 | 4 | 95 |

### Beispiel Nr. 10

### Bemisia tabaci (BEMITA Spritzbehandlung)

| | | |
|---|---|---|
| Lösungsmittel: | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblattscheiben *(Gossypium hirsutum),* die von Larven der Weißen Fliege *(Bemisia tabaci)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Weiße Fliegen abgetötet wurden; 0 % bedeutet, dass keine Weiße Fliege abgetötet wurde.

Bei diesem Test zeigen z. B. die folgenden Verbindungen der Herstellungsbeispiele gute Wirksamkeit: siehe Tabelle

| Beispiel | Wirkstoffkonzentration in ppm | Abtötungsgrad in % nach 7 Tagen |
|---|---|---|
| Beispiel 13 | 500 | 94 |

### Beispiel Nr. 11

### Ctenocephalides felis; oral (CTECFE)

| | |
|---|---|
| Lösungsmittel: | Dimethylsulfoxid |

Zwecks Herstellung einer zweckmäßigen Wirkstoffzubereitung vermischt man 1 Gewichtsteil Wirkstoff mit der angegebenen Menge Wasser. Ein Teil des Konzentrats wird mit citiriertem Rinderblut verdünnt und die gewünschte Konzentration hergestellt.
20 nüchterne adulte Flöhe *(Ctenocephalides felis)* werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann. Während das Blut auf 37° C erwärmt wird, ist im Bereich der Flohkammern Raumtemperatur.

Nach der gewünschten Zeit wird die Abtötung in % bestimmt. Dabei bedeutet 100 %, dass alle Flöhe abgetötet wurden; 0 % bedeutet, dass kein Floh abgetötet wurde.

Bei diesem Test zeigen z.B. die folgenden Verbindungen der Herstellungsbeispiele eine gute Wirkung: siehe Tabelle

| Beispiel | Wirkstoffkonzentration in ppm | Abtötungsgrad in % nach 2 Tagen |
|---|---|---|
| Beispiel 13 | 100 | 80 |
| Beispiel 15 | 100 | 90 |

### Beispiel Nr. 12

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele gute Wirksamkeit: siehe Tabelle

| Beispiel | Wirkstoffkonzentration in ppm | Abtötungsgrad in % nach 2 Tagen |
|---|---|---|
| Beispiel 11 | 100 | 100 |
| Beispiel 13 | 100 | 100 |
| Beispiel 15 | 100 | 90 |
| Beispiel 8 | 100 | 100 |

### Beispiel Nr. 13

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach der gewünschten Zeit wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele gute Wirksamkeit: siehe Tabelle

| Beispiel | Wirkstoffkonzentration in µg/Tier | Abtötungsgrad in % nach 7 Tagen |
|---|---|---|
| Beispiel 11 | 20 | 100 |

### Biologische Vergleichsbeispiele

### Beispiel Nr. 1

### Myzus persicae -Test (MYZUPE T)

| | | |
|---|---|---|
| Lösungsmittel: | 7 | Gewichtsteile Dimethylformamid |
| | | |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea),* die stark von der Grünen Pfirsichblattlaus *(Myzus persicae)* befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeipiele überlegende Wirksamkeit gegenüber dem Stand der Technik: siehe Tabelle

| Beispiel | Wirkstoffkonzentration in ppm | Abtötungsgrad in % nach 6 Tagen |
|---|---|---|
| Beispiel 85 ^{a}) | 20 | 15 |
| Beispiel 86^{a)} | 20 | 50 |
| Beispiel 4 | 20 | 80 |
| Beispiel 85 ^{a}) | 20 | 15 |
| Beispiel 1 | 20 | 95 |

| | | |
|---|---|---|
| ^{a)} vgl. EP 0539588 A1 | | |

### Beispiel Nr. 2

### Myzus-Test, (MYZUPE Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (Brassica pekinensis), die von allen Stadien der Grünen Pfirsichblattlaus (Myzus persicae) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle

| Beispiel | Wirkstoffkonzentration in g/ha | Abtötungsgrad in % nach 5 Tagen |
|---|---|---|
| Beispiel 87 ^{a}) | 100 | 0 |
| Beispiel 9 | 100 | 90 |
| Beispiel 176 ^{a)} | 4 | 0 |
| Beispiel 16 | 4 | 70 |
| Beispiel 89 ^{a)} | 4 | 0 |
| Beispiel 1 | 4 | 80 |

| | | |
|---|---|---|
| ^{a)} vgl. EP 0539588 A1 | | |

### Beispiel Nr. 3

### Myzus persicae -Test, hydroponische Behandlung (MYZUPE sys.)

| | | |
|---|---|---|
| Lösungsmittel: | 7 | Gewichtsteile Dimethylformamid |
| | | |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Wasser gemischt. Die angegebene Konzentration bezieht sich auf die Wirkstoffmenge pro Volumeneinheit Wasser (mg/1= ppm). Man füllt das behandelte Wasser in Gefäße mit einer Erbsenpflanze *(Pisum sativum),* anschließend wird mit der Grünen Pfirsichblattlaus *(Myzus persicae)* infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle

| Beispiel | Wirkstoffkonzentration in ppm | Abtötungsgrad in % nach 6 Tagen |
|---|---|---|
| Beispiel 86 ^{a)} | 0,8 0,16 | 75 0 |
| Beispiel 4 | 0,8 0,16 | 100 55 |
| Beispiel 85 ^{a)} | 0,8 | 40 |
| Beispiel 1 | 0,8 | 100 |

| | | |
|---|---|---|
| ^{a)} vgl. EP 0539588 A1 | | |

### Beispiel Nr. 4

### Myzus persicae -Test (MYZUPE)

| | | |
|---|---|---|
| Lösungsmittel: | 7 | Gewichtsteile Dimethylformamid |
| | | |
| Emulgator: | 2 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen oder Ammoniumsalzen und Penetrationsförderer wird die entsprechende Menge nach dem Verdünnen jeweils der fertigen Präparatelösung zupipettiert.

Paprikapflanzen *(Capsicum annuum),* die stark von der Grünen Pfirsichblattlaus *(Myzus persicaei)* befallen sind, werden durch Sprühen mit der Wirkstoffzubereitung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

### Beispiel Nr. 5

### Myzus-Test; oral; (MYZUPE O)

| | | |
|---|---|---|
| Lösungsmittel: | 80 | Gewichtsteile Aceton |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit allen Stadien der Grünen Pfirsichblattlaus *(Myzus persicae)* besetzt, durch saugen an der Wirkstoffzubereitung der gewünschten Konzentration wird behandelt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle

| Beispiel | Wirkstoffkonzentration in ppm | Abtötungsgrad in % nach 5 Tagen |
|---|---|---|
| Beispiel 85 ^{a)} | 0,032 | 0 |
| Beispiel 1 | 0,032 | 100 |

| | | |
|---|---|---|
| ^{a)} vgl. EP 0539588 A1 | | |

### Beispiel Nr. 6

### Aphis gossypii -Test (APHIGO)

| | | |
|---|---|---|
| Lösungsmittel: | 7 | Gewichtsteile Dimethylformamid |
| | | |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter *(Gossypium hirsutum),* die stark von der Baumwollblattlaus *(Aphis gossypii)* befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindungen der Herstellungsbeispiele gute Wirksamkeit: siehe Tabelle

| Beispiel | Wirkstoffkonzentration in ppm | Abtötungsgrad in % nach 6 Tagen |
|---|---|---|
| Beispiel 85 ^{a)} | 4 | 45 |
| Beispiel 86 ^{a)} | 4 | 10 |
| Beispiel 4 | 4 | 99 |
| Beispiel 85 ^{a)} | 4 | 45 |
| | 0,8 | 15 |
| Beispiel 1 | 4 | 98 |
| | 0,8 | 60 |

| | | |
|---|---|---|
| ^{a)} vgl. EP 0539588 A1 | | |

### Beispiel Nr. 7

### Meloidogyne-Test (MELGIN Spritzbehandlung)

| | | |
|---|---|---|
| Lösungsmittel: | 80 | Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, Meloidogyne incognita-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele überlegene Wirksamkeit gegenüber dem Stand der Technik: siehe Tabelle

| Beispiel | Wirkstoffkonzentration in ppm | Abtötungsgrad in % nach 14 Tagen |
|---|---|---|
| Beispiel 85 ^{a)} | 20 | 0 |
| Beispiel 4 | 20 | 70 |

| | | |
|---|---|---|
| ^{a)} vgl. EP 0539588 A1 | | |

### Beispiel Nr. 8

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele überlegene Wirksamkeit gegenüber dem Stand der Technik: siehe Tabelle

| Beispiel | Wirkstoffkonzentration in ppm | Abtötungsgrad in % nach 2 Tagen |
|---|---|---|
| Beispiel 85 ^{a)} | 4 | 45 |
| Beispiel 1 | 4 | 100 |

| | | |
|---|---|---|
| ^{a)} vgl. EP 0539588 A1 | | |

### Beispiel Nr. 9

### Ctenocephalides felis; oral (CTECFE)

| | |
|---|---|
| Lösungsmittel: | Dimethylsulfoxid |

Zwecks Herstellung einer zweckmäßigen Wirkstoffzubereitung vermischt man 1 Gewichtsteil Wirkstoff mit der angegebenen Menge Wasser. Ein Teil des Konzentrats wird mit citiriertem Rinderblut verdünnt und die gewünschte Konzentration hergestellt.
20 nüchterne adulte Flöhe *(Ctenocephalides felis)* werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann. Während das Blut auf 37° C erwärmt wird, ist im Bereich der Flohkammern Raumtemperatur.

Nach der gewünschten Zeit wird die Abtötung in % bestimmt. Dabei bedeutet 100 %, dass alle Flöhe abgetötet wurden; 0 % bedeutet, dass kein Floh abgetötet wurde.

Bei diesem Test zeigen z. B. die folgenden Verbindungen der Herstellungsbeispiele überlegene Wirksamkeit gegenüber dem Stand der Technik: siehe Tabelle

| Beispiel | Wirkstoffkonzentration in ppm | Abtötungsgrad in % nach 1 Tagen |
|---|---|---|
| Beispiel 85 ^{a)} | 100 | 0 |
| Beispiel 86 ^{a)} | 100 | 0 |
| Beispiel 4 | 100 | 30 |

| | | |
|---|---|---|
| ^{a)} vgl. EP 0539588 A1 | | |

### Beispiel Nr. 10

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele überlegene Wirksamkeit gegenüber dem Stand der Technik: siehe Tabelle

| Beispiel | Wirkstoffkonzentration in ppm | Abtötungsgrad in % nach 7 Tagen |
|---|---|---|
| Beispiel 85 ^{a)} | 20 | 0 |
| Beispiel 86 ^{a)} | 20 | 0 |
| Beispiel 4 | 20 | 100 |

| | | |
|---|---|---|
| ^{a)} vgl. EP 0539588 A1 | | |

## Patentansprüche

1. Verwendung einer Verbindung der Formel (I) 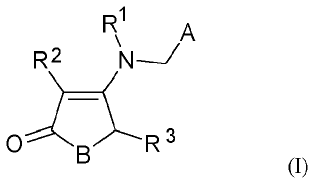 in welcher
A für Pyrid-3-yl welches in 6-Position durch Fluor, Chlor, Brom, Methyl oder Trifluormethyl substituiert ist oder für 2-Chlor-pyrazin-5-yl oder für 2-Chlor-1,3-thiazol-5-yl steht,
B für Sauerstoff, Schwefel oder Methylen steht,
R¹ für Halogen-C₁₋₃-alkyl, Halogen-C₂₋₃-alkenyl, Halogencyclopropyl,
R² für Wasserstoff oder Halogen steht und
R³ für Wasserstoff oder Methyl steht,
zur Bekämpfung von Insekten, Spinnentieren und Nematoden, die in den Forsten, im Vorrats- und Materialschutz vorkommen.

2. Verwendung gemäß Anspruch 1, wobei in der Verbindung der Formel (I)
A für 6-Brom-pyrid-3-yl steht,
B für Sauerstoff steht
R¹ für 2 Fluorethyl steht,
R² für Wasserstoff steht und
R³ für Wasserstoff steht.

3. Verwendung gemäß Anspruch 1, wobei in der Verbindung der Formel (I)
A für 6-Fluor-pyrid-3-yl steht,
B für Sauerstoff steht
R¹ für 2,2-Difluorethyl steht,
R² für Wasserstoff steht und
R³ für Wasserstoff steht.

4. Verwendung gemäß Anspruch 1, wobei in der Verbindung der Formel (I)
A für 6-Chlor-pyrid-3-yl steht,
B für Sauerstoff steht
R¹ für 2,2-Difluorethyl steht,
R² für Chlor steht und
R³ für Wasserstoff steht.

5. Verwendung gemäß Anspruch 1, wobei in der Verbindung der Formel (I)
A für 6-Brom-pyrid-3-yl steht,
B für Sauerstoff steht
R¹ für 2,2-Difluorethyl steht,
R² für Wasserstoff steht und
R³ für Wasserstoff steht.

6. Verwendung gemäß Anspruch 1, wobei in der Verbindung der Formel (I)
A für 6-Chlor-pyrid-3-yl steht,
B für Sauerstoff steht
R¹ für 2-Fluorethyl steht,
R² für Wasserstoff steht und
R³ für Wasserstoff steht.

7. Verwendung gemäß Anspruch 1, wobei in der Verbindung der Formel (I)
A für 6-Chlor-pyrid-3-yl steht,
B für Sauerstoff steht
R¹ für 2,2-Difluorethyl steht,
R² für Wasserstoff steht und
R³ für Wasserstoff steht.

8. Verwendung gemäß Anspruch 1, wobei in der Verbindung der Formel (I)
A für 2-Chlor-1,3-thiazol-5-yl steht,
B für Sauerstoff steht
R¹ für 2-Fluorethyl steht,
R² für Wasserstoff steht und
R³ für Wasserstoff steht.

9. Verwendung wie in einem der Ansprüche 1 bis 8 definiert, wobei die Verbindung als Mittel vorliegt, das einen Gehalt von mindestens einer Verbindung der Formel (I) gemäß einem der Ansprüche 1 bis 8 und übliche Streckmitteln und/oder oberflächenaktive Substanzen enthält.

10. Verwendung wie in einem der Ansprüche 1 bis 9 definiert, wobei man zur Bekämpfung der Insekten, Spinnentieren und Nematoden eine Verbindung der Formel (I) gemäß einem der Ansprüche 1 bis 8 auf die Insekten, Spinnentieren und Nematoden und/oder ihren Lebensraum einwirken lässt.

11. Verwendung einer Verbindung der Formel (1) gemäß einem der Ansprüche 1 bis 9, wobei die Verbindung der Formel (I) in seinen handelsüblichen Formulierungen sowie in den aus diesen Formulierungen bereiteten Anwendungsformen in Mischung mit Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegt.

12. Verwendung einer Verbindung der Formel (I) wie einem der Ansprüche 1 bis 9 definiert, für die Herstellung eines Medikaments zur Bekämpfung von Ekto- und Endoparasiten.

13. Verwendung einer Verbindung der Formel (I) wie einem der Ansprüche 1 bis 9 definiert, als Pflanzenschutzmittel zur Bekämpfung von Insekten Spinnentieren und Nematoden die in der Landwirtschaft, im Gartenbau, in Forsten, in Gärten und Freizeiteinrichtungen, und im Vorrats- und Materialschutz vorkommen.

## Claims

1. Use of a compound of the formula (I) 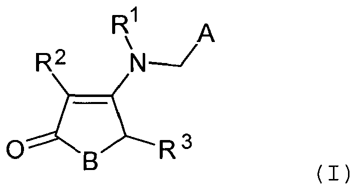 in which
A represents pyrid-3-yl which is substituted in the 6-position by fluorine, chlorine, bromine, methyl or trifluoromethyl or represents 2-chloropyrazin-5-yl or represents 2-chloro-1,3-thiazol-5-yl,
B represents oxygen, sulphur or methylene,
R¹ represents halo-C₁₋₃-alkyl, halo-C₂₋₃-alkenyl, halocyclopropyl,
R² represents hydrogen or halogen and
R³ represents hydrogen or methyl,
for controlling insects, arachnids and nematodes which are encountered in forests and in the protection of stored products and of materials.

2. Use according to Claim 1 where, in the compound of the formula (I),
A represents 6-bromopyrid-3-yl,
B represents oxygen
R¹ represents 2-fluoroethyl,
R² represents hydrogen and
R³ represents hydrogen.

3. Use according to Claim 1 where, in the compound of the formula (I),
A represents 6-fluoropyrid-3-yl,
B represents oxygen
R¹ represents 2,2-difluoroethyl,
R² represents hydrogen and
R³ represents hydrogen.

4. Use according to Claim 1 where, in the compound of
the formula (I),
A represents 6-chloropyrid-3-yl,
B represents oxygen
R¹ represents 2,2-difluoroethyl,
R² represents chlorine and
R³ represents hydrogen.

5. Use according to Claim 1 where, in the compound of the formula (I),
A represents 6-bromopyrid-3-yl,
B represents oxygen
R¹ represents 2,2-difluoroethyl,
R² represents hydrogen and
R³ represents hydrogen.

6. Use according to Claim 1 where, in the compound of the formula (I),
A represents 6-chloropyrid-3-yl,
B represents oxygen
R¹ represents 2-fluoroethyl,
R² represents hydrogen and
R³ represents hydrogen.

7. Use according to Claim 1 where, in the compound of the formula (I),
A represents 6-chloropyrid-3-yl,
B represents oxygen
R¹ represents 2,2-difluoroethyl,
R² represents hydrogen and
R³ represents hydrogen.

8. Use according to Claim 1 where, in the compound of the formula (I),
A represents 2-chloro-1,3-thiazol-5-yl,
B represents oxygen
R¹ represents 2-fluoroethyl,
R² represents hydrogen and
R³ represents hydrogen.

9. Use as defined in any of Claims 1 to 8, where the compound is present as a composition which comprises at least one compound of the formula (I) according to any of Claims 1 to 8 and customary extenders and/or surfactants.

10. Use as defined in any of Claims 1 to 9, where, for controlling the insects, arachnids and nematodes, a compound of the formula (I) according to any of Claims 1 to 8 is allowed to act on the insects, arachnids and nematodes and/or their habitat.

11. Use of a compound of the formula (I) according to any of Claims 1 to 9, where the compound of the formula (I) is present in its commercial formulations and in the use forms prepared from these formulations as a mixture with insecticides, attractants, sterilizing agents, bactericides, acaricides, nematicides, fungicides, growth-regulating substances, herbicides, safeners, fertilizers or semiochemicals.

12. Use of a compound of the formula (I) as defined in any of Claims 1 to 9 for preparing a medicament for controlling ecto- and endoparasites.

13. Use of a compound of the formula (I) as defined in any of Claims 1 to 9 as crop protection agent for controlling insects, arachnids and nematodes which are encountered in agriculture, in horticulture, in forests, in gardens and in leisure facilities, and in the protection of stored products and of materials.

## Revendications

1. Utilisation d'un composé de formule (I) 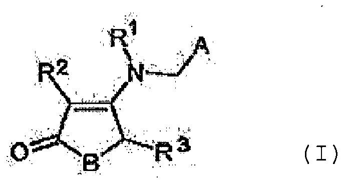 dans laquelle
A représente un groupe pyrid-3-yle qui est substitué en position 6 par fluoro, chloro, bromo, méthyle ou trifluorométhyle ou représente le groupe 2-chloro-pyrazin-5-yle ou 2-chloro-1,3-thiazol-5-yle,
B représente un atome d'oxygène, de soufre ou le groupe méthylène,
R¹ représente un groupe halogéno-alkyle(C₁-C₃), halogéno-alcényle(C₂-C₃), halogénocyclopropyle,
R² représente un atome d'hydrogène ou d'halogène et
R³ représente un atome d'hydrogène ou le groupe méthyle,
pour la lutte pour la lutte contre des insectes, arachnides et nématodes qui se rencontrent dans les
or forêts, dans la protection des stocks et des matériaux.

2. Utilisation selon la revendication 1, dans laquelle dans le composé de formule (I)
A représente le groupe 6-bromo-pyrid-3-yle,
B représente un atome d'oxygène,
R¹ représente le groupe 2-fluoroéthyle,
R² représente un atome d'hydrogène et
R³ représente un atome d'hydrogène.

3. Utilisation selon la revendication 1, dans laquelle dans le composé de formule (I)
A représente le groupe 6-fluoro-pyrid-3-yle,
B représente un atome d'oxygène,
R¹ représente le groupe 2,2-difluoroéthyle,
R² représente un atome d'hydrogène et
R³ représente un atome d'hydrogène.

4. Utilisation selon la revendication 1, dans laquelle dans le composé de formule (I)
A représente le groupe 6-chloro-pyrid-3-yle,
B représente un atome d'oxygène,
R¹ représente le groupe 2,2-difluoroéthyle,
R² représente un atome de chlore et
R³ représente un atome d'hydrogène.

5. Utilisation selon la revendication 1, dans laquelle dans le composé de formule (I)
A représente le groupe 6-bromo-pyrid-3-yle,
B représente un atome d'oxygène,
R¹ représente le groupe 2,2-difluoroéthyle,
R² représente un atome d'hydrogène et
R³ représente un atome d'hydrogène.

6. Utilisation selon la revendication 1, dans laquelle dans le composé de formule (I)
A représente le groupe 6-chloro-pyrid-3-yle,
B représente un atome d'oxygène,
R¹ représente le groupe 2-fluoroéthyle,
R² représente un atome d'hydrogène et
R³ représente un atome d'hydrogène.

7. Utilisation selon la revendication 1, dans laquelle dans le composé de formule (I)
A représente le groupe 6-chloro-pyrid-3-yle,
B représente un atome d'oxygène,
R¹ représente le groupe 2,2-difluoroéthyle,
R² représente un atome d'hydrogène et
R³ représente un atome d'hydrogène.

8. Utilisation selon la revendication 1, dans laquelle dans le composé de formule (I)
A représente le groupe 2-chloro-1,3-thiazol-5-yle,
B représente un atome d'oxygène,
R¹ représente le groupe 2-fluoroéthyle,
R² représente un atome d'hydrogène et
R³ représente un atome d'hydrogène.

9. Utilisation telle que définie dans l'une quelconque des revendications 1 à 8, dans laquelle le composé se trouve sous forme d'une composition qui contient au moins un composé de formule (I) selon l'une quelconque des revendications 1 à 8 et des excipients usuels et/ou des substances tensioactives usuelles.

10. Utilisation telle que définie dans l'une quelconque des revendications 1 à 9, dans laquelle pour la lutte contre des insectes, des arachnides et des nématodes on fait agir sur les insectes, arachnides et nématodes et/ou leur habitat un composé de formule (I) selon l'une quelconque des revendications 1 à 8.

11. Utilisation d'un composé de formule (I) selon l'une quelconque des revendications 1 à 9, dans laquelle le composé de formule (I) est présent dans ses formulations usuelles du commerce ainsi que dans les formes d'utilisation préparées à partir de ces formulations en mélange avec des insecticides, attractants, stérilisants, bactéricides, acaricides, nématicides, fongicides, régulateurs de croissance, herbicides, phytoprotecteurs, fertilisants ou substances sémiochimiques.

12. Utilisation d'un composé de formule (I) tel que défini dans l'une quelconque des revendications 1 à 9, pour la fabrication d'un médicament destiné à la lutte contre des ecto- et endoparasites.

13. Utilisation d'un composé de formule (I) tel que défini dans l'une quelconque des revendications 1 à 9, en tant qu'agent phytosanitaire pour la lutte contre des insectes, arachnides et nématodes qui se rencontrent en agriculture, en horticulture, dans des forêts, dans des jardins et des installations de loisirs, et dans la protection des stocks et des matériaux.
